# EUROPEAN PATENT APPLICATION

(11) **EP 3 978 476 A1**
(43) Date of publication of application: **06.04.2022**
(21) Application number: 20815328.8
(22) Date of filing: 21.05.2020
(51) Int. Cl.: C07D 215/227, A61K 31/47, A61P 1/16, A61P 43/00

(54) **SOLID FORM OF AROMATIC COMPOUND AND PREPARATION METHOD THEREFOR**

(30) Priority: 24.05.2019 CN 201910448548
(71) Applicant: Sichuan Kelun-Biotech Biopharmaceutical Co., Ltd., Chengdu, Sichuan 611138 (CN)
(72) Inventor: YI, Shidong, Chengdu, Sichuan 611138 (CN); WANG, Tianming, Chengdu, Sichuan 611138 (CN); WU, Zhuan, Chengdu, Sichuan 611138 (CN); LI, Long, Chengdu, Sichuan 611138 (CN); YANG, Chengxi, Chengdu, Sichuan 611138 (CN); SONG, Zhiquan, Chengdu, Sichuan 611138 (CN); LONG, Dong, Chengdu, Sichuan 611138 (CN); TIAN, Qiang, Chengdu, Sichuan 611138 (CN); SONG, Hongmei, Chengdu, Sichuan 611138 (CN); XUE, Tongtong, Chengdu, Sichuan 611138 (CN); WANG, Jingyi, Chengdu, Sichuan 611138 (CN)
(74) Representative: Mewburn Ellis LLP
(86) International application number: PCT/CN2020/091471
(87) International publication number: WO 2020/238740

(57) **Abstract**

The present application relates to a solid form of a compound of Formula (I), a method for preparing the solid form, a pharmaceutical composition comprising the solid form, and uses of the solid form in preparing a medicament for treating or preventing peroxisome proliferator-activated receptor (PPAR)-related diseases such as nonalcoholic fatty liver disease (NAFLD).

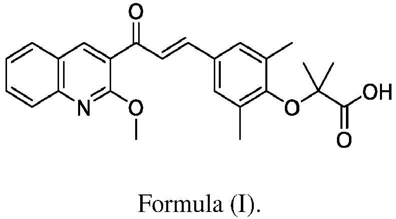

## Description

The present application is based on and claims the benefit of priority from Chinese application No. 201910448548.5, filed on May 24, 2019, the disclosures of which are incorporated herein by reference in its entirety.

### Technical Field

The present application relates to a crystalline or amorphous form of (*E*)*-*2-(4-(3-(2-methoxyquinolin-3-yl)-3-oxo-1-propen-1-yl)-2,6-dimethylphenoxy)-2-methyl-propionic acid (hereinafter referred to as "the compound of Formula (I)") and a method for preparing the same, a pharmaceutical composition comprising the same, and use thereof in manufacture of a medicament for prevention or treatment of a disease or condition such as non-alcoholic fatty liver disease (NAFLD).

### Background Art

Non-alcoholic fatty liver disease (NAFLD) is a type of clinical pathological syndrome with hepatic histopathological changes similar to alcoholic liver disease, but without a history of excessive drinking, including simple fatty liver (SFL), non-alcoholic steatohepatitis (NASH) and related cirrhosis, in which NASH is an important intermediate stage of NAFLD progression. With the high incidence of insulin resistance and its related multiple metabolic syndrome, the prevalence of NAFLD/NASH has gradually increased. At present, NASH has become one of the important precursor lesions of cirrhosis second only to chronic viral hepatitis and alcoholic liver disease, and is a common cause of abnormal serum transaminase in healthy people. The effective prevention and treatment of NASH is expected to prevent the progression of chronic liver disease and reduce the occurrence of liver cirrhosis as well as liver disease-related disability and death. Non-alcoholic fatty liver disease is a new challenge in the contemporary medical field, and the development of drugs for the treatment of non-alcoholic fatty liver-related diseases has important clinical significance. Therefore, there is an urgent need for effective and safe drugs for the treatment of non-alcoholic fatty liver-related diseases.

In the international patent application PCT/CN2018/115615 filed by the applicant, phenoxyacetic acid compounds with novel structure are disclosed, and these compounds as peroxisome proliferator-activated receptor (PPAR) agonists have good pharmacokinetic and pharmacodynamic properties in vivo and in vitro, and have the potential to prevent or treat diseases or conditions such as non-alcoholic fatty liver disease (NAFLD). Therefore, further research and development of such compounds and obtaining their crystalline or amorphous forms suitable for the manufacture of pharmaceutical preparations are urgently needed in the field of medicine.

### Contents of the Disclosure

One aspect of the present application provides a crystalline form of the compound of Formula (I) (E)-2-(4-(3-(2-methoxyquinolin-3-yl)-3-oxo-1-propen-1-yl)-2,6-dimethylphenoxy)-2-methyl-propionic acid, especially crystalline form A, B, C, D or E, and an amorphous form of the compound of Formula (I).

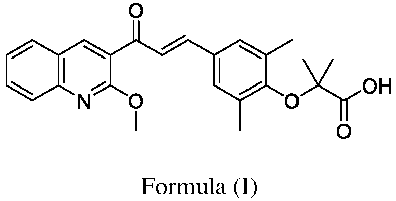

Another aspect of the present application provides a method for preparing crystalline form A, B, C, D, E or amorphous form of the compound of Formula (I), which comprises reacting any solid form of the compound of Formula (I) with an inorganic acid or organic acid to precipitate a solid, and then separating and drying the precipitated solid. The method for precipitating a solid comprises, but is not limited to, method of gas-solid infiltration, method of anti-solvent crystallization, method of suspending and stirring at room temperature, method of suspending and stirring at high temperature, method of gas-liquid infiltration, method of slow evaporation at room temperature, method of slow cooling, and the like.

Another aspect of the present application provides a pharmaceutical composition, which comprises a crystalline form of the compound of Formula (I), especially crystalline form A, B, C, D or E, or amorphous form of the compound of Formula (I), or any combination thereof, and one or more pharmaceutically acceptable carriers.

Another aspect of the present application provides a method for treating a disease or condition associated with peroxisome proliferator-activated receptor (PPAR) in an individual, which comprises administering to an individual in need thereof a therapeutically effective amount of a crystalline form of the compound of Formula (I), especially crystalline form A, B, C, D or E, or amorphous form of the compound of Formula (I), or any combination thereof.

Another aspect of the present application provides a crystalline form of the compound of Formula (I), especially crystalline form A, B, C, D or E, or amorphous form of the compound of Formula (I), or any combination thereof, for use in the treatment of a disease or condition associated with peroxisome proliferator-activated receptor (PPAR) in an individual.

Another aspect of the present application provides use of a crystalline form of the compound of Formula (I), especially crystalline form A, B, C, D or E, or amorphous form of the compound of Formula (I), or any combination thereof in the manufacture of a medicament for the treatment of a disease or condition associated with peroxisome proliferator-activated receptor (PPAR) in an in dividual.

The crystalline form of the compound of Formula (I) of the present application has one or more of the following advantageous properties:
i) High solubility, high dissolution rate, low hygroscopicity, high fluidity or significantly improved viscosity.
ii) Excellent physical and chemical stability, including but not limited to light stability, thermal stability, high humidity resistance, etc. For example, good light stability can ensure the reliability of the crystalline form during storage and transportation, thereby ensuring the safety of the preparation, can make the crystalline form not need to be specially packaged to prevent the influence of light, thereby reducing the cost, can prevent the crystalline form from being degraded due to the influence of light, thereby improving the safety of the preparation and the effectiveness after long-term storage, and can make the patients who take the crystalline form not worry that the photosensitivity of the preparation due to exposure to sunlight. Good thermal stability enables the crystals to remain stable for a long time, and to be suitable for standard preparation production processes. Good physical and chemical stability make the crystalline form easy to be prepared and more suitable to product preparations.
iii) Improved metabolism, increased bioavailability, reduced toxicity or increased safety.
iv) Suitable and convenient for production in large scale with saved cost.

### Brief Description of the Drawings

Figure 1: XRPD pattern of crystalline form A of the compound of Formula (I);
Figure 2: DSC curve of crystalline form A of the compound of Formula (I);
Figure 3: TGA curve of crystalline form A of the compound of Formula (I);
Figure 4: XRPD pattern of crystalline form B of the compound of Formula (I);
Figure 5: DSC curve of crystalline form B of the compound of Formula (I);
Figure 6: TGA curve of crystalline form B of the compound of Formula (I);
Figure 7: XRPD pattern of crystalline form C of the compound of Formula (I);
Figure 8: DSC curve of crystalline form C of the compound of Formula (I);
Figure 9: TGA curve of crystalline form C of the compound of Formula (I);
Figure 10: XRPD pattern of crystalline form D of the compound of Formula (I);
Figure 11: DSC curve of crystalline form D of the compound of Formula (I);
Figure 12: TGA curve of crystalline form D of the compound of Formula (I);
Figure 13: XRPD pattern of crystalline form E of the compound of Formula (I);
Figure 14: DSC curve of crystalline form E of the compound of Formula (I);
Figure 15: TGA curve of crystalline form E of the compound of Formula (I);
Figure 16: XRPD pattern of amorphous form of the compound of Formula (I).

### Detailed Description of the Disclosure

### Definitions

Unless otherwise defined below, the meanings of all technical terms and scientific terms used herein are intended to be the same as those commonly understood by those skilled in the art. The mention of the technology used herein is intended to refer to the technology generally understood in the art including those technical changes or equivalent technology substitutions that are obvious to those skilled in the art. Although it is believed that the following terms are well understood by those skilled in the art, the following definitions are still set forth to better explain the present application.

As used herein, the terms "comprising", "including", "having", "containing" or "involving" and other variants herein are inclusive or open-ended, and do not exclude other unlisted elements, methods or steps.

As used herein, the term "about" refers to that the stated value is within an acceptable standard error, such as ±0.05, ±0.1, ±0.2, ±0.3, ±1, ±2, or ±3 etc., as understood by those of ordinary skill in the art.

As used herein, the term "solid form" includes all solid-state forms of the compound of Formula (I) and salts of the compound of Formula (I), such as crystalline forms or amorphous forms.

As used herein, the term "amorphous" refers to any solid substance exhibiting unordered in three dimensions. In some cases, an amorphous solid can be characterized by known techniques including XRPD crystallography, solid state nuclear magnetic resonance (ssNMR) spectroscopy, differential scanning calorimetry (DSC), or some combinations of these techniques. As explained below, an amorphous solid has a diffuse XRPD pattern, which usually includes one or two broad peaks (i.e., peaks with a base width with 2θ of about 5° or greater).

As used herein, the term "crystalline form" or "crystal" refers to any solid substance exhibiting ordered in three dimensions, as opposed to an amorphous solid substance, which has a characteristic XRPD pattern with well-defined peaks.

As used herein, the term "X-ray powder diffraction pattern (XRPD pattern)" refers to an experimentally observed diffraction pattern or parameters derived therefrom. The XRPD pattern is usually characterized by peak positions (abscissa) and/or peak intensities (ordinate).

As used herein, the term "2θ" refers to a peak position expressed in degrees experimentally set in an X-ray diffraction experiment, and is usually a unit of abscissa in a diffraction pattern. If the reflection is diffracted when an incident beam forms an angle θ with a certain lattice plane, it is set in the experiment that the reflected beam needs to be recorded at an angle of 2θ. It should be understood that a specific 2θ value of a specific crystalline form mentioned herein is intended to mean an 2θ value (expressed in degrees) measured using the X-ray diffraction experimental conditions described herein.

As used herein, the term "thermogravimetric analysis (TGA) curve" refers to a curve recorded by a thermogravimetric analyzer.

As used herein, the term "differential scanning calorimetry (DSC) curve" refers to a curve recorded by a differential scanning calorimeter.

As used herein, the term "substantially the same" for X-ray diffraction peak positions means that variations of position and intensity of representative peak are taken into account. For example, those skilled in the art will understand that a peak position (2θ) will show some variations, usually as much as 0.1 to 0.2 degrees, and the instrument used to measure diffraction will also show some variations. In addition, those skilled in the art will understand that the relative peak intensity will show variations between instruments and changes due to the degree of crystallinity, the preferred orientation, the surface of prepared sample, and other factors known to those skilled in the art, and should be regarded as merely qualitative measurement. Similarly, as used herein, the term "substantially the same" for DSC curve and TGA curve is also intended to cover the variations related to the analysis techniques as known to those skilled in the art.

As used herein, the term "good solvent" refers to a solvent used to dissolve the compound (I) or a salt of the compound (I) of the present application.

As used herein, the term "anti-solvent" refers to a solvent for reducing the solubility of a substance to be crystallized in a good solvent.

As used herein, the term "anti-solvent crystallization method" refers to a method for reducing the solubility of a substance to be crystallized in the good solvent by using a good solvent in combination with an anti-solvent. According to the order of solvent addition, the anti-solvent crystallization method can be divided into the anti-solvent addition method and the reversed anti-solvent addition method. The anti-solvent addition method refers to a method in which a substance to be crystallized is dissolved in a good solvent and then an anti-solvent is added thereto, thereby precipitating a crystal; while the reversed anti-solvent addition method refers to a method in which a substance to be crystallized is dissolved in a good solvent, and then the resulting solution is added into an anti-solvent, thereby precipitating a crystal.

As used herein, the term "hydrocarbons" refers to, for example, hydrocarbons having 1 to 10 carbon atoms, which include alkanes, halogenated alkanes, alkenes, alkynes, and aromatic hydrocarbons, including but not limited to dichloromethane, trichloromethane (chloroform), n-hexane, n-heptane and toluene.

As used herein, the term "alcohols" refers to, for example, alcohols having 1 to 10 carbon atoms, which include, but not limited to, methanol, ethanol, 1-propanol (n-propanol), 2-propanol (isopropanol), 1-butanol, 2-butanol and tert-butanol.

As used herein, the term "ethers" as used herein refers to, for example, ethers having 2 to 6 carbon atoms, including chain ethers and cyclic ethers (such as furans (including tetrahydrofurans) and dioxanes), specifically including but not limited to diethyl ether, diisopropyl ether, methyl tert-butyl ether, tetrahydrofuran, 2-methyltetrahydrofuran, dioxane, cyclopentyl methyl ether, anisole and dimethoxyethane.

As used herein, the term "nitriles" refers to, for example, nitriles having 2 to 6 carbon atoms, which include, but are not limited to, acetonitrile and propionitrile.

As used herein, the term "ketones" refers to, for example, ketones having 2 to 6 carbon atoms, which include, but are not limited to, acetone, butanone, methyl ethyl ketone, methyl isobutyl ketone, and diethyl ketone.

As used herein, the term "esters" refers to, for example, esters having 3 to 10 carbon atoms, which include, but are not limited to, ethyl acetate, propyl acetate, isopropyl acetate, ethyl isopropate, dimethyl carbonate and butyl acetate.

As used herein, the term "organic acids" refers to, for example, organic acids having 1 to 10 carbon atoms, which include, but are not limited to, formic acid and acetic acid.

As used herein, the term "sulfones" refers to, for example, sulfones or sulfoxides having 2 to 10 carbon atoms, which include, but are not limited to, dimethylsulfoxide.

As used herein, the term "amides" refers to, for example, amides having 1 to 10 carbon atoms, which include, but are not limited to, dimethylformamide or dimethylacetamide.

As used herein, the term "nitrogen-containing heterocycles" refers to, for example, nitrogen-containing heterocycles having 3 to 10 carbon atoms and at least one nitrogen atom, which includes, but is not limited to, N-methylpyrrolidone.

As used herein, the numerical range (such as "1 to 10") and its sub-ranges (such as "2 to 10", "2 to 6", "3 to 10"), etc., cover any number of the numerical range (for example, 1, 2, 3, 4, 5, 6, 7, 8, 9 or 10), and is not limited to integers.

In the present application, solid line ( 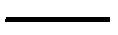 ), wavy line ( 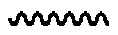 ), solid wedge ( 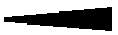 ), or broken wedge ( 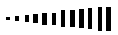 ) can be used herein to describe a chemical bond of the compound of the present application. The solid line to be used to depict a bond to an asymmetric atom is intended to indicate that all possible stereoisomers at the position of the atom are included (e.g., specific enantiomers, racemic mixtures, etc.). The wavy line to be used to depict a bond to an asymmetric atom is intended to indicate that the bond is either a solid wedge ( 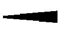 ) bond or a broken wedge ( 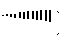 ) bond. The solid or broken wedge to be used to depict a bond to an asymmetric atom is intended to indicate that there is the stereoisomer as shown. When present in a racemic mixture, the solid and broken wedges is used to define relative stereochemistry, rather than absolute stereochemistry.

It should be understood that different types of equipment or different test conditions may give slightly different DSC curve. For example, Mettler Toledo DSC1 differential scanning calorimeter can be used to measure DSC curve. As used herein, the term "substantially the same" for DSC curve takes into account the position of representative characteristic peak. For example, those skilled in the art will understand that the position of characteristic peak will show some variation, usually as much as 5°C. For solid samples with polymorphs, the heating rate of DSC test has a greater influence on DSC curve. At a faster heating rate, the thermal hysteresis effect of instrument is obvious, and the solid crystalline form with high melting point is too late to recrystallize, so that DSC curve often only shows the melting endothermic peak of the crystalline form with low melting point. At a moderate heating rate, DSC curve shows two peaks: the melting endothermic peak of the crystalline form with low melting point and the melting endothermic peak of the crystalline form with high melting point; and only at a lower heating rate, the thermal hysteresis effect of instrument is weaker, there will be 3 peaks: the melting peak of the crystalline form with low melting point, the exothermic peak of recrystallization, the melting endothermic peak of the crystalline form with high melting point. The skilled person will understand that the determination of the heating rate range corresponding to the above-mentioned different DSC curve will vary depending on the weight, shape, particle size and distribution of the test sample (Reference: Giron D. Thermal analysis and calorimetric methods in the characterisation of polymorphs and solvates [J]. Thermochimica Acta, 1995, 248:1-59.).

The solid form of the compound of Formula (I), the salt of the compound of Formula (I), or the crystalline form thereof can be recovered by a method, including decantation, centrifugation, evaporation, gravity filtration, suction filtration, or any other methods used for recovering solid under pressure or under reduced pressure. The recovered solid can optionally be dried. The "Drying" in the present application is carried out under reduced pressure (preferably vacuum) until the residual solvent content is reduced to the limit range given in the guidelines of International Conference on Harmonisation of Technical Requirements for Registration of Pharmaceuticals for Human Use ("ICH"). The residual solvent content depends on the type of solvent, but does not exceed about 5000 ppm, or preferably about 4000 ppm, or more preferably about 3000 ppm. The drying can be performed in a tray dryer, a vacuum oven, an air oven, a cone vacuum dryer, a rotary vacuum dryer, a fluidized bed dryer, a spin flash dryer, a rapid dryer, etc. The drying can be performed at a temperature of less than about 100°C, less than about 80°C, less than about 60°C, less than about 50°C, less than about 30°C, or any other suitable temperature, under atmospheric pressure or reduced pressure (preferably under vacuum) for any desired time (e.g., about 1, 2, 3, 5, 10, 15, 20, 24 hours, or overnight) that can achieve the desired result, as long as the quality of the product does not deteriorate. The drying can be performed for any desired number of times until the desired product quality is achieved. The dried product can optionally undergo a comminution operation to produce the desired particle size. The product can be ground or micronized before or after drying. Techniques that can be used to reduce particle size include, but are not limited to, ball milling, roller milling, as well as hammer milling, and jet milling.

Crystalline forms and amorphous form of the compound of Formula (I) and a method for preparing the same

Crystalline form A of the compound of Formula (I)

An object of the present application is to provide a crystalline form A of the compound of Formula (I) as shown below:

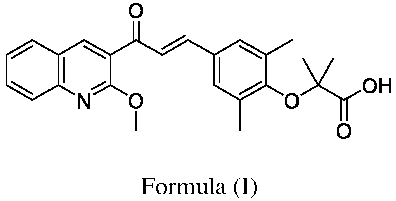

According to an embodiment of the present application, the present application provides the crystalline form A of the compound of Formula (I). The crystalline form A of the compound of Formula (I) has an XRPD pattern as measured by using Cu-Ka radiation and expressed in 2θ angles, which comprises characteristic peaks at diffraction angles (2θ) of 11.1±0.2°, 11,5±0.2°, 16.2±0.2°, 17.0±0.2°, 19.0±0.2° and 24.9±0.2°. In some embodiments, the crystalline form A of the compound of Formula (I) has an XRPD pattern as measured by using Cu-Ka radiation and expressed in 2θ angles, which comprises characteristic peaks at diffraction angles (2θ) of 7.3±0.2°, 11.1±0.2°, 11.5±0.2°, 16.0±0.2°, 16.2±0.2°, 16.9±0.2°, 19.0±0.2°, 20.9±0.2°, and 24.9±0.2°. In some embodiments, the crystalline form A of the compound of Formula (I) has an XRPD pattern as measured by using Cu-Ka radiation and expressed in 2θ angles, which comprises characteristic peaks at diffraction angles (2θ) of 7.3±0.2°, 8.2±0.2°, 11.1±0.2°, 11.5±0.2°, 11.9±0.2°, 16.0±0.2°, 16.2±0.2°, 16.9±0.2°, 19.0±0.2°, 20.9±0.2°, 24.0±0.2° and 24.9±0.2°. In some embodiments, the crystalline form A of the compound of Formula (I) has an XRPD pattern as measured by using Cu-Ka radiation and expressed in 2θ angles, which comprises peaks at diffraction angles (2θ) as follows:

| 2θ (°) ± 0.2° | 2θ (°) ± 0.2° | 2θ (°) ± 0.2° |
|---|---|---|
| 7.26 | 21.11 | 30.72 |
| 8.20 | 21.97 | 31.05 |
| 10.16 | 22.39 | 31.56 |
| 11.12 | 23.04 | 31.72 |
| 11.52 | 23.22 | 31.87 |
| 11.75 | 23.42 | 32.46 |
| 11.89 | 23.96 | 32.72 |
| 12.98 | 24.17 | 33.41 |
| 13.63 | 24.39 | 33.44 |
| 14.60 | 24.91 | 33.91 |
| 15.22 | 25.24 | 34.33 |
| 15.70 | 25.55 | 34.44 |
| 16.04 | 25.84 | 35.13 |
| 16.15 | 26.20 | 35.44 |
| 16.52 | 27.02 | 35.72 |
| 16.96 | 27.45 | 36.36 |
| 17.79 | 27.55 | 36.85 |
| 18.47 | 28.01 | 37.50 |
| 19.03 | 28.36 | 38.09 |
| 20.44 | 28.67 | 38.43 |
| 20.59 | 29.36 | 38.94 |
| 20.78 | 29.88 | 39.25 |
| 20.94 | 30.49 | - |

In some embodiments, the crystalline form A of the compound of Formula (I) has an XRPD pattern as measured by using Cu-Ka radiation and expressed in 2θ angles, which comprises peaks at diffraction angles (2θ) as follows:

| 2θ (°)± 0.2° | Intensity, % | 2θ (°)± 0.2° | Intensity, % |
|---|---|---|---|
| 7.26 | 13.24 | 25.55 | 2.62 |
| 8.20 | 5.71 | 25.84 | 6.43 |
| 10.16 | 2.34 | 26.20 | 2.54 |
| 11.12 | 65.90 | 27.02 | 15.51 |
| 11.52 | 81.34 | 27.45 | 1.09 |
| 11.75 | 19.21 | 27.55 | 21.88 |
| 11.89 | 28.37 | 28.01 | 0.67 |
| 12.98 | 17.53 | 28.36 | 3.21 |
| 13.63 | 8.39 | 28.67 | 15.64 |
| 14.60 | 3.70 | 29.36 | 0.90 |
| 15.22 | 3.27 | 29.88 | 0.64 |
| 15.70 | 14.33 | 30.49 | 3.27 |
| 16.04 | 35.21 | 30.72 | 2.81 |
| 16.15 | 100.00 | 31.05 | 2.87 |
| 16.52 | 25.02 | 31.56 | 0.96 |
| 16.96 | 83.21 | 31.72 | 3.28 |
| 17.79 | 1.00 | 31.87 | 3.07 |
| 18.47 | 21.66 | 32.46 | 1.15 |
| 19.03 | 93.62 | 32.72 | 6.10 |
| 20.44 | 6.63 | 33.41 | 0.56 |
| 20.59 | 10.46 | 33.44 | 7.90 |
| 20.78 | 11.29 | 33.91 | 4.93 |
| 20.94 | 32.98 | 34.33 | 2.42 |
| 21.11 | 19.69 | 34.44 | 2.16 |
| 21.97 | 6.94 | 35.13 | 3.04 |
| 22.39 | 4.38 | 35.44 | 0.18 |
| 23.04 | 13.75 | 35.72 | 2.23 |
| 23.22 | 15.19 | 36.36 | 5.22 |
| 23.42 | 2.26 | 36.85 | 0.23 |
| 23.96 | 24.06 | 37.50 | 0.44 |
| 24.17 | 21.31 | 38.09 | 1.08 |
| 24.39 | 3.75 | 38.43 | 0.11 |
| 24.91 | 72.14 | 38.94 | 2.06 |
| 25.24 | 4.53 | 39.25 | 1.44 |

In some embodiments, the crystalline form A of the compound of Formula (I) has an XRPD pattern as measured by using Cu-Ka radiation and expressed in 2θ angles, which comprises peaks at diffraction angles (2θ) as follows:

| 2θ (°) ± 0.2° | Interplanar distance, d(Å) | Intensity, % | 2θ (°) ± 0.2° | Interplanar distance, d(Å) | Intensity, % |
|---|---|---|---|---|---|
| 7.26 | 12.17 | 13.24 | 25.55 | 3.48 | 2.62 |
| 8.20 | 10.77 | 5.71 | 25.84 | 3.45 | 6.43 |
| 10.16 | 8.70 | 2.34 | 26.20 | 3.40 | 2.54 |
| 11.12 | 7.95 | 65.90 | 27.02 | 3.30 | 15.51 |
| 11.52 | 7.68 | 81.34 | 27.45 | 3.25 | 1.09 |
| 11.75 | 7.52 | 19.21 | 27.55 | 3.23 | 21.88 |
| 11.89 | 7.44 | 28.37 | 28.01 | 3.18 | 0.67 |
| 12.98 | 6.81 | 17.53 | 28.36 | 3.14 | 3.21 |
| 13.63 | 6.49 | 8.39 | 28.67 | 3.11 | 15.64 |
| 14.60 | 6.06 | 3.70 | 29.36 | 3.04 | 0.90 |
| 15.22 | 5.82 | 3.27 | 29.88 | 2.99 | 0.64 |
| 15.70 | 5.64 | 14.33 | 30.49 | 2.93 | 3.27 |
| 16.04 | 5.52 | 35.21 | 30.72 | 2.91 | 2.81 |
| 16.15 | 5.48 | 100.00 | 31.05 | 2.88 | 2.87 |
| 16.52 | 5.36 | 25.02 | 31.56 | 2.83 | 0.96 |
| 16.96 | 5.22 | 83.21 | 31.72 | 2.82 | 3.28 |
| 17.79 | 4.98 | 1.00 | 31.87 | 2.81 | 3.07 |
| 18.47 | 4.80 | 21.66 | 32.46 | 2.76 | 1.15 |
| 19.03 | 4.66 | 93.62 | 32.72 | 2.73 | 6.10 |
| 20.44 | 4.34 | 6.63 | 33.41 | 2.68 | 0.56 |
| 20.59 | 4.31 | 10.46 | 33.44 | 2.68 | 7.90 |
| 20.78 | 4.27 | 11.29 | 33.91 | 2.64 | 4.93 |
| 20.94 | 4.24 | 32.98 | 34.33 | 2.61 | 2.42 |
| 21.11 | 4.21 | 19.69 | 34.44 | 2.60 | 2.16 |
| 21.97 | 4.04 | 6.94 | 35.13 | 2.55 | 3.04 |
| 22.39 | 3.97 | 4.38 | 35.44 | 2.53 | 0.18 |
| 23.04 | 3.86 | 13.75 | 35.72 | 2.51 | 2.23 |
| 23.22 | 3.83 | 15.19 | 36.36 | 2.47 | 5.22 |
| 23.42 | 3.80 | 2.26 | 36.85 | 2.44 | 0.23 |
| 23.96 | 3.71 | 24.06 | 37.50 | 2.40 | 0.44 |
| 24.17 | 3.68 | 21.31 | 38.09 | 2.36 | 1.08 |
| 24.39 | 3.65 | 3.75 | 38.43 | 2.34 | 0.11 |
| 24.91 | 3.57 | 72.14 | 38.94 | 2.31 | 2.06 |
| 25.24 | 3.53 | 4.53 | 39.25 | 2.29 | 1.44 |

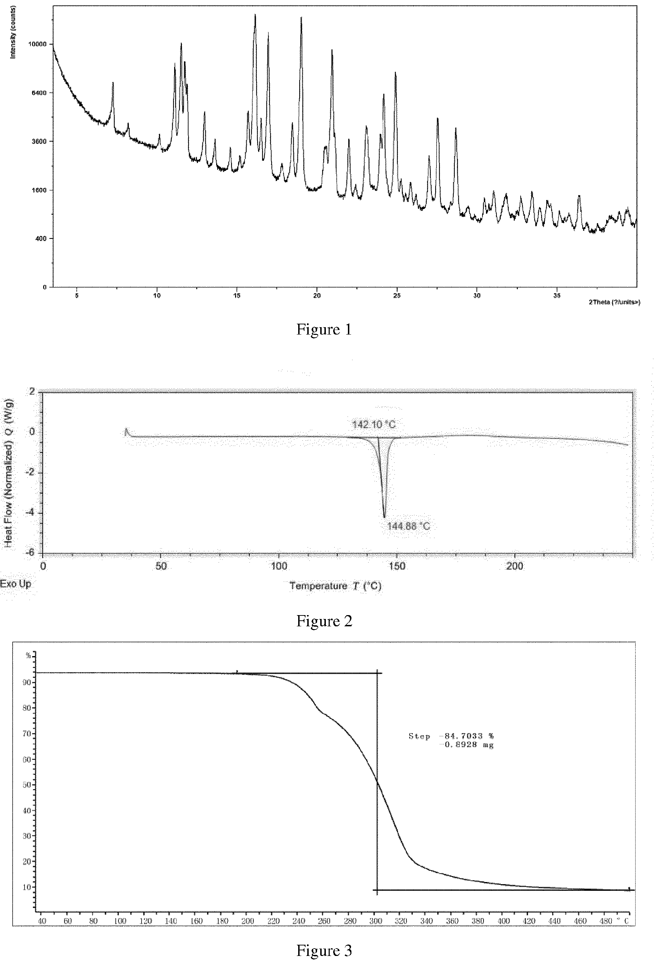

In some embodiments, the crystalline form A of the compound of Formula (I) has an XRPD pattern as measured by using Cu-Ka radiation and expressed in 2θ angles, which comprises peaks at the diffraction angles (2θ) substantially the same as those shown in Figure 1. In some embodiments, the crystalline form A of the compound of Formula (I) has XRPD peak positions as measured by using Cu-Ka radiation and expressed in 2θ angles, which are substantially the same as those shown in Figure 1. In some embodiments, the crystalline form A of the compound of Formula (I) has an XRPD pattern which is substantially the same as that shown in Figure 1.

In some embodiments, the crystalline form A of the compound of Formula (I) of the present application has a DSC curve which comprises a characteristic peak at about 142±5°C (initial temperature). In some embodiments, the crystalline form A of the compound of Formula (I) has a DSC curve which comprises an endothermic peak in the range of about 130°C to 160°C. In some embodiments, the crystalline form A of the compound of Formula (I) has a DSC curve which comprises a characteristic peak at substantially the same temperature as shown in Figure 2. In some embodiments, the crystalline form A of the compound of Formula (I) has a DSC curve which has a characteristic peak position that is substantially the same as those shown in Figure 2.

In some embodiments, the crystalline form A of the compound of Formula (I) has a TGA curve which is substantially the same as that shown in Figure 3.

In some embodiments, the crystalline form A of the compound of Formula (I) of the present application is a non-solvate. In some embodiments, the crystalline form A of the compound of Formula (I) of the present application is an anhydrous crystal.

### Crystalline form B of the compound of Formula (I)

According to an embodiment of the present application, the present application provides a crystalline form B of the compound of Formula (I). The crystalline form B of the compound of Formula (I) has an XRPD pattern as measured by using Cu-Ka radiation and expressed in 2θ angles, which comprises characteristic peaks at diffraction angles (2θ) of 7.5±0.2°, 10.8±0.2°, 12.4±0.2°, 14.7±0.2°, 18.2±0.2°, and 25.3±0.2°. In some embodiments, the crystalline form B of the compound of Formula (I) has an XRPD pattern as measured by using Cu-Ka radiation and expressed in 2θ angles, which comprises characteristic peaks at diffraction angles (2θ) of 7.5±0.2°, 10.8±0.2°, 12.4±0.2°, 14.7±0.2°, 14.8±0.2°, 15.0±0.2°, 16.4±0.2°, 17.1±0.2°, 18.2±0.2°, 20.9±0.2°, 21.2±0.2°, 23.4±0.2° and 25.3±0.2°. In some embodiments, the crystalline form B of the compound of Formula (I) has an XRPD pattern as measured by using Cu-Ka radiation and expressed in 2θ angles, which comprises characteristic peaks at diffraction angles (2θ) of 7.5±0.2°, 10.8±0.2°, 12.4±0.2°, 14.7± 0.2°, 14.8±0.2°, 15.0±0.2°, 16.4±0.2°, 17.1±0.2°, 18.2±0.2°, 19.2±0.2°, 20.1±0.2°, 20.9±0.2°, 21.2±0.2°, 23.4±0.2°, 25.3±0.2°, 28.5±0.2°, 30.3±0.2°, 33.8±0.2°, 35.8±0.2° and 36.8±0.2°. In some embodiments, the crystalline form B of the compound of Formula (I) has an XRPD pattern as measured by using Cu-Ka radiation and expressed in 2θ angles, which comprises peaks at diffraction angles (2θ) as follows:

| 2θ (°) ± 0.2° | 2θ (°) ± 0.2° | 2θ (°) ± 0.2° |
|---|---|---|
| 7.53 | 20.14 | 30.10 |
| 10.84 | 20.88 | 30.27 |
| 12.37 | 21.24 | 31.47 |
| 14.75 | 21.88 | 32.25 |
| 14.87 | 22.57 | 33.84 |
| 15.51 | 23.44 | 34.89 |
| 16.41 | 24.87 | 35.24 |
| 17.07 | 25.33 | 35.79 |
| 18.18 | 26.23 | 36.80 |
| 19.19 | 27.68 | 37.39 |
| 19.39 | 28.51 | - |

In some embodiments, the crystalline form B of the compound of Formula (I) has an XRPD pattern as measured by using Cu-Ka radiation and expressed in 2θ angles, which comprises peaks at diffraction angles (2θ) as follows:

| 2θ (°)± 0.2° | Intensity, % | 2θ (°)± 0.2° | Intensity, % |
|---|---|---|---|
| 7.53 | 30.72 | 23.44 | 33.27 |
| 10.84 | 64.96 | 24.87 | 7.00 |
| 12.37 | 97.13 | 25.33 | 53.29 |
| 14.75 | 76.59 | 26.23 | 8.84 |
| 14.87 | 17.87 | 27.68 | 16.34 |
| 15.51 | 68.91 | 28.51 | 24.56 |
| 16.41 | 30.76 | 30.10 | 9.16 |
| 17.07 | 90.10 | 30.27 | 29.56 |
| 18.18 | 100.00 | 31.47 | 13.69 |
| 19.19 | 19.99 | 32.25 | 4.21 |
| 19.39 | 9.41 | 33.84 | 39.08 |
| 20.14 | 20.38 | 34.89 | 5.85 |
| 20.88 | 14.52 | 35.24 | 2.80 |
| 21.24 | 42.86 | 35.79 | 14.91 |
| 21.88 | 17.98 | 36.80 | 15.90 |
| 22.57 | 9.51 | 37.39 | 2.88 |

In some embodiments, the crystalline form B of the compound of Formula (I) has an XRPD pattern as measured by using Cu-Ka radiation and expressed in 2θ angles, which comprises peaks at diffraction angles (2θ) as follows:

| 2θ (°) ± 0.2° | Interplanar distance, d(Å) | Intensity, % | 2θ (°) ± 0.2° | Interplanar distance, d(Å) | Intensity, % |
|---|---|---|---|---|---|
| 7.53 | 11.73 | 30.72 | 23.44 | 3.79 | 33.27 |
| 10.84 | 8.15 | 64.96 | 24.87 | 3.58 | 7.00 |
| 12.37 | 7.15 | 97.13 | 25.33 | 3.51 | 53.29 |
| 14.75 | 6.00 | 76.59 | 26.23 | 3.39 | 8.84 |
| 14.87 | 5.95 | 17.87 | 27.68 | 3.22 | 16.34 |
| 15.51 | 5.71 | 68.91 | 28.51 | 3.13 | 24.56 |
| 16.41 | 5.40 | 30.76 | 30.10 | 2.97 | 9.16 |
| 17.07 | 5.19 | 90.10 | 30.27 | 2.95 | 29.56 |
| 18.18 | 4.88 | 100.00 | 31.47 | 2.84 | 13.69 |
| 19.19 | 4.62 | 19.99 | 32.25 | 2.77 | 4.21 |
| 19.39 | 4.57 | 9.41 | 33.84 | 2.65 | 39.08 |
| 20.14 | 4.41 | 20.38 | 34.89 | 2.57 | 5.85 |
| 20.88 | 4.25 | 14.52 | 35.24 | 2.54 | 2.80 |
| 21.24 | 4.18 | 42.86 | 35.79 | 2.51 | 14.91 |
| 21.88 | 4.06 | 17.98 | 36.80 | 2.44 | 15.90 |
| 22.57 | 3.94 | 9.51 | 37.39 | 2.40 | 2.88 |

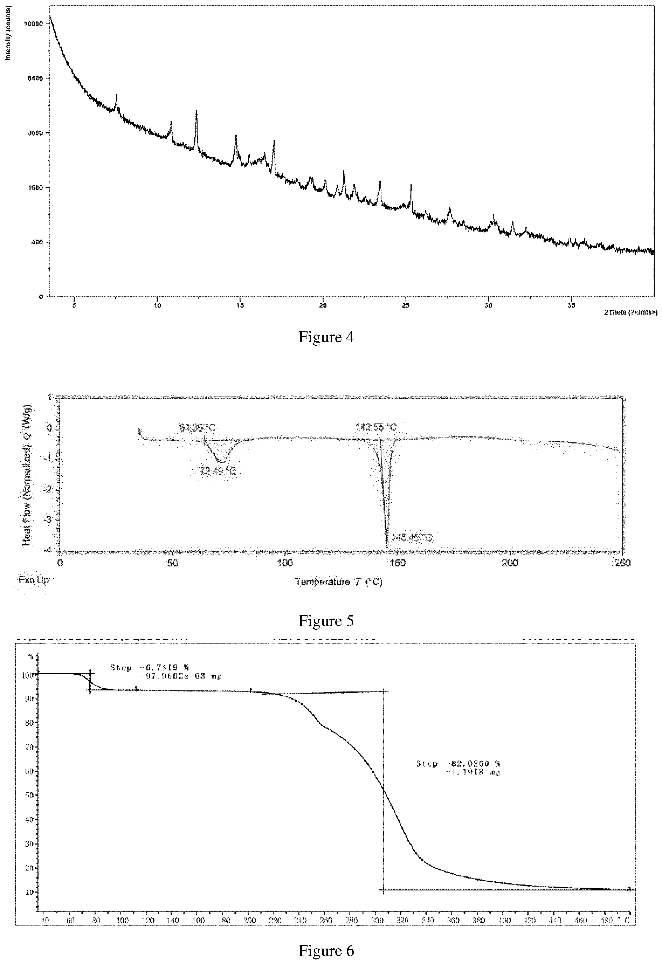

In some embodiments, the crystalline form B of the compound of Formula (I) has an XRPD pattern as measured by using Cu-Ka radiation and expressed in 2θ angles, which comprises peaks at the diffraction angles (2θ) substantially the same as those shown in Figure 4. In some embodiments, the crystalline form B of the compound of Formula (I) has XRPD peak positions as measured by using Cu-Ka radiation and expressed in 2θ angles, which are substantially the same as those shown in Figure 4.

In some embodiments, the crystalline form B of the compound of Formula (I) of the present application has a DSC curve which comprises endothermic peaks at about 64±5°C and 142±5°C (initial temperature). In some embodiments, the crystalline form B of the compound of Formula (I) has a DSC curve which comprises endothermic peaks in the range of about 55°C to 80°C and the range of 130°C to 150°C. In some embodiments, the crystalline form B of the compound of Formula (I) has a DSC curve which comprises characteristic peaks at substantially the same temperatures as shown in Figure 5. In some embodiments, the crystalline form B of the compound of Formula (I) has a DSC curve which has characteristic peak positions that are substantially the same as those shown in Figure 5.

In some embodiments, the crystalline form B of the compound of Formula (I) of the present application has a thermogravimetric analysis (TGA) curve which has a weight loss of 5% to 8% at about 60±10°C, preferably has a weight loss of 6% to 7%, more preferably has a weight loss of 6.7%. In some embodiments, the crystalline form B of the compound of Formula (I) has a TGA curve which comprises the temperature substantially the same as that shown in Figure 6 at which a weight loss occurs. In some embodiments, the crystalline form B of the compound of Formula (I) has a TGA curve which is substantially the same as that shown in Figure 6.

In some embodiments, the crystalline form B of the compound of Formula (I) of the present application is an ether solvate, wherein the molar ratio of the compound of Formula (I) to the solvent is 1:0.5.

### Crystalline form C of the compound of Formula (I)

According to one embodiment of the present application, the present application provides a crystalline form C of the compound of Formula (I). The crystalline form C of the compound of Formula (I) has a XRPD pattern as measured by using Cu-Ka radiation and expressed in 2θ angles, which comprises characteristic peaks at the diffraction angles (2θ) of 7.8±0.2°, 14.6±0.2°, 17.1±0.2°, 21.6±0.2° and 22.7±0.2°. In some embodiments, the crystalline form C of the compound of Formula (I) has a XRPD pattern as measured by using Cu-Ka radiation and expressed in 2θ angles, which comprises characteristic peaks at the diffraction angles (2θ) of 7.8±0.2°, 14.6±0.2°, 15.6±0.2°, 17.1±0.2°, 20.4±0.2°, 20.6±0.2°, 21.6±0.2°, 22.7±0.2°, 29.3±0.2°, 29.6±0.2° and 31.3±0.2°. In some embodiments, the crystalline form C of the compound of Formula (I) has a XRPD pattern as measured by using Cu-Ka radiation and expressed in 2θ angles, which comprises characteristic peaks at the diffraction angles (2θ) of 7.8±0.2°, 9.6±0.2°, 10.8±0.2°, 12.7± 0.2°, 13.1±0.2°, 14.6±0.2°, 15.6±0.2°, 17.1±0.2°, 18.6±0.2°, 20.4±0.2°, 20.6±0.2°, 21.1±0.2°, 21.6±0.2°, 22.3±0.2°, 22.7±0.2°, 23.6±0.2°, 27.2±0.2°, 27.8±0.2°, 28.7±0.2°, 29.3±0.2°, 29.6±0.2° and 31.3±0.2°. In some embodiments, the crystalline form C of the compound of Formula (I) has a XRPD pattern as measured by using Cu-Ka radiation and expressed in 2θ angles, which comprises peaks at the diffraction angles (2θ) as follows:

| 2θ (°) ± 0.2° | 2θ (°) ± 0.2° | 2θ (°) ± 0.2° |
|---|---|---|
| 7.77 | 22.33 | 30.22 |
| 9.62 | 22.67 | 30.93 |
| 10.78 | 23.57 | 31.27 |
| 12.68 | 23.76 | 31.66 |
| 13.11 | 24.04 | 32.03 |
| 14.63 | 24.57 | 32.23 |
| 15.64 | 25.30 | 32.92 |
| 16.47 | 25.50 | 33.56 |
| 17.06 | 25.67 | 34.24 |
| 18.59 | 25.93 | 34.55 |
| 19.88 | 27.23 | 35.44 |
| 20.30 | 27.81 | 36.06 |
| 20.43 | 28.69 | 36.51 |
| 20.87 | 29.31 | 37.51 |
| 21.05 | 29.33 | 37.83 |
| 21.58 | 29.58 | 38.60 |

In some embodiments, the crystalline form C of the compound of Formula (I) has an XRPD pattern as measured by using Cu-Ka radiation and expressed in 2θ angles, which comprises peaks at diffraction angles (2θ) as follows:

| 2θ (°) ± 0.2° | Intensity, % | 2θ (°)± 0.2° | Intensity, % | 2θ (°) ± 0.2° | Intensity, % |
|---|---|---|---|---|---|
| 7.77 | 26.37 | 22.33 | 1.76 | 30.22 | 1.36 |
| 9.62 | 5.81 | 22.67 | 34.18 | 30.93 | 2.81 |
| 10.78 | 8.02 | 23.57 | 10.41 | 31.27 | 13.38 |
| 12.68 | 3.61 | 23.76 | 2.84 | 31.66 | 2.50 |
| 13.11 | 1.52 | 24.04 | 3.69 | 32.03 | 2.88 |
| 14.63 | 47.86 | 24.57 | 1.82 | 32.23 | 3.78 |
| 15.64 | 17.92 | 25.30 | 1.02 | 32.92 | 0.39 |
| 16.47 | 5.36 | 25.50 | 2.14 | 33.56 | 0.51 |
| 17.06 | 100.00 | 25.67 | 1.55 | 34.24 | 0.82 |
| 18.59 | 7.05 | 25.93 | 0.93 | 34.55 | 3.83 |
| 19.88 | 2.42 | 27.23 | 3.67 | 35.44 | 1.62 |
| 20.30 | 9.12 | 27.81 | 4.03 | 36.06 | 2.07 |
| 20.43 | 11.94 | 28.69 | 3.41 | 36.51 | 2.62 |
| 20.87 | 15.76 | 29.31 | 16.03 | 37.51 | 1.71 |
| 21.05 | 9.45 | 29.33 | 1.62 | 37.83 | 1.30 |
| 21.58 | 38.08 | 29.58 | 12.67 | 38.60 | 1.60 |

In some embodiments, the crystalline form C of the compound of Formula (I) has an XRPD pattern as measured by using Cu-Ka radiation and expressed in 2θ angles, which comprises peaks at diffraction angles (2θ) as follows:

| 2θ (°) ± 0.2° | Interplanar distance, d(Å) | Intensity, % | 2θ (°) ± 0.2° | Interplanar distance, d(Å) | Intensity, % |
|---|---|---|---|---|---|
| 7.77 | 11.38 | 26.37 | 25.67 | 3.47 | 1.55 |
| 9.62 | 9.19 | 5.81 | 25.93 | 3.43 | 0.93 |
| 10.78 | 8.20 | 8.02 | 27.23 | 3.27 | 3.67 |
| 12.68 | 6.98 | 3.61 | 27.81 | 3.21 | 4.03 |
| 13.11 | 6.75 | 1.52 | 28.69 | 3.11 | 3.41 |
| 14.63 | 6.05 | 47.86 | 29.31 | 3.04 | 16.03 |
| 15.64 | 5.66 | 17.92 | 29.33 | 3.04 | 1.62 |
| 16.47 | 5.38 | 5.36 | 29.58 | 3.02 | 12.67 |
| 17.06 | 5.19 | 100.00 | 30.22 | 2.95 | 1.36 |
| 18.59 | 4.77 | 7.05 | 30.93 | 2.89 | 2.81 |
| 19.88 | 4.46 | 2.42 | 31.27 | 2.86 | 13.38 |
| 20.30 | 4.37 | 9.12 | 31.66 | 2.82 | 2.50 |
| 20.43 | 4.34 | 11.94 | 32.03 | 2.79 | 2.88 |
| 20.87 | 4.25 | 15.76 | 32.23 | 2.78 | 3.78 |
| 21.05 | 4.22 | 9.45 | 32.92 | 2.72 | 0.39 |
| 21.58 | 4.11 | 38.08 | 33.56 | 2.67 | 0.51 |
| 22.33 | 3.98 | 1.76 | 34.24 | 2.62 | 0.82 |
| 22.67 | 3.92 | 34.18 | 34.55 | 2.59 | 3.83 |
| 23.57 | 3.77 | 10.41 | 35.44 | 2.53 | 1.62 |
| 23.76 | 3.74 | 2.84 | 36.06 | 2.49 | 2.07 |
| 24.04 | 3.70 | 3.69 | 36.51 | 2.46 | 2.62 |
| 24.57 | 3.62 | 1.82 | 37.51 | 2.40 | 1.71 |
| 25.30 | 3.52 | 1.02 | 37.83 | 2.38 | 1.30 |
| 25.50 | 3.49 | 2.14 | 38.60 | 2.33 | 1.60 |

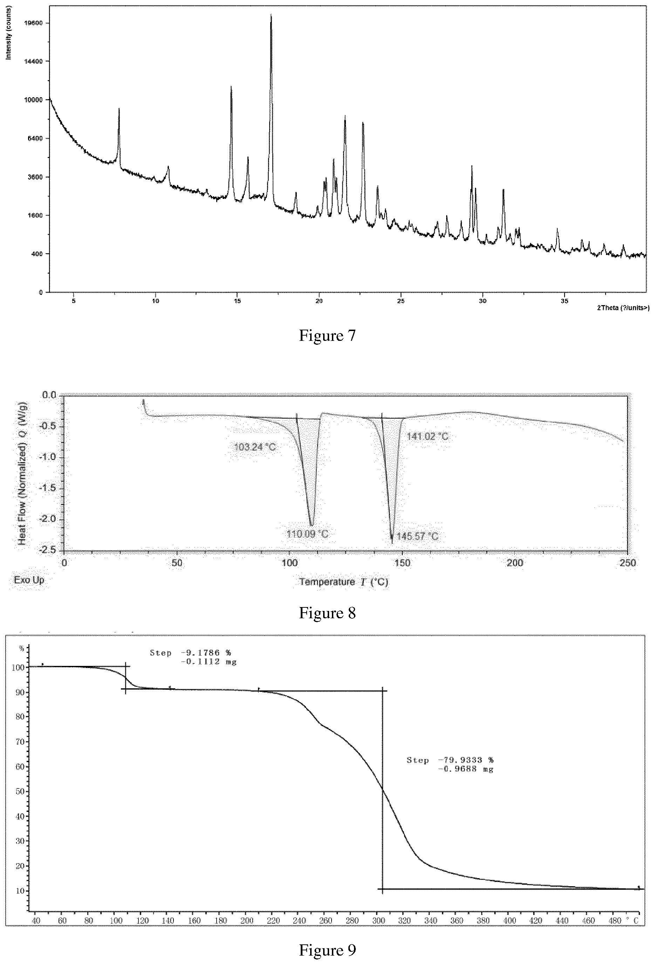

In some embodiments, the crystalline form C of the compound of Formula (I) has an XRPD pattern as measured by using Cu-Ka radiation and expressed in 2θ angles, which comprises peaks at the diffraction angles (2θ) substantially the same as those shown in Figure 7. In some embodiments, the crystalline form C of the compound of Formula (I) has XRPD peak positions as measured by using Cu-Ka radiation and expressed in 2θ angles, which are substantially the same as those shown in Figure 7.

In some embodiments, the crystalline form C of the compound of Formula (I) of the present application has a DSC curve which comprises endothermic peaks at about 103±5°C and 141±5°C (initial temperature). In some embodiments, the crystalline form C of the compound of Formula (I) has a DSC curve which comprises endothermic peaks in the range of about 80°C to 115°C and the range of 130°C to 150°C. In some embodiments, the crystalline form C of the compound of Formula (I) has a DSC curve which comprises characteristic peaks at substantially the same temperatures as shown in Figure 8. In some embodiments, the crystalline form C of the compound of Formula (I) has a DSC curve which has characteristic peak positions that are substantially the same as those shown in Figure 8.

In some embodiments, the crystalline form C of the compound of Formula (I) of the present application has a thermogravimetric analysis (TGA) curve which has a weight loss of 7% to 11 % at about 80°C~100°C, preferably has a weight loss of 8% to 10%, more preferably has a weight loss of 9.2%. In some embodiments, the crystalline form C of the compound of Formula (I) has a TGA curve which comprises the temperature substantially the same as that shown in Figure 9 at which a weight loss occurs. In some embodiments, the crystalline form C of the compound of Formula (I) has a TGA curve which is substantially the same as that shown in Figure 9.

In some embodiments, the crystalline form C of the compound of Formula (I) of the present application is an ethyl acetate solvate, wherein the molar ratio of the compound of Formula (I) to the solvent is 1:0.5.

### Crystalline form D of the compound of Formula (I)

According to one embodiment of the present application, the present application provides a crystalline form D of the compound of Formula (I). The crystalline form D of the compound of Formula (I) has a XRPD pattern as measured by using Cu-Ka radiation and expressed in 2θ angles, which comprises characteristic peaks at the diffraction angles (2θ) of 10.8±0.2°, 14.6±0.2°, 14.7±0.2°, 20.3±0.2°, 21.4±0.2°, 26.5±0.2°, and 31.2±0.2°. In some embodiments, the crystalline form D of the compound of Formula (I) has a XRPD pattern as measured by using Cu-Ka radiation and expressed in 2θ angles, which comprises characteristic peaks at the diffraction angles (2θ) of 10.8±0.2°, 13.1±0.2°, 14.6±0.2°, 14.7±0.2°, 17.0±0.2°, 20.1±0.2°, 20.3±0.2°, 20.5±0.2°, 20.7±0.2°, 21.0±0.2°, 21.4±0.2°, 26.5±0.2° and 31.2±0.2°. In some embodiments, the crystalline form D of the compound of Formula (I) has a XRPD pattern as measured by using Cu-Ka radiation and expressed in 2θ angles, which comprises characteristic peaks at the diffraction angles (2θ) of 7.6±0.2°, 10.8±0.2°, 13.1±0.2°, 14.6±0.2°, 14.7±0.2°, 15.4±0.2°, 16.1±0.2°, 17.0±0.2°, 19.9±0.2°, 20.1±0.2°, 20.3±0.2°, 20.5±0.2°, 20.7±0.2°, 21.0±0.2°, 21.4±0.2°, 22.5±0.2°, 23.3±0.2°, 23.5±0.2°, 26.5±0.2°, 30.2±0.2°, 31.2±0.2° and 32.1±0.2°. In some embodiments, the crystalline form D of the compound of Formula (I) has a XRPD pattern as measured by using Cu-Ka radiation and expressed in 2θ angles, which comprises peaks at the diffraction angles (2θ) as follows:

| 2θ (°) ± 0.2° | 2θ (°) ± 0.2° | 2θ (°) ± 0.2° |
|---|---|---|
| 7.65 | 20.30 | 26.47 |
| 10.76 | 20.50 | 27.48 |
| 13.12 | 20.73 | 28.03 |
| 14.56 | 20.98 | 30.20 |
| 14.68 | 21.36 | 31.16 |
| 15.39 | 22.48 | 32.06 |
| 16.15 | 23.25 | 32.85 |
| 16.96 | 23.52 | 34.76 |
| 19.50 | 24.69 | 35.20 |
| 19.86 | 25.27 | 37.35 |
| 20.10 | 26.02 | - |

In some embodiments, the crystalline form D of the compound of Formula (I) has an XRPD pattern as measured by using Cu-Ka radiation and expressed in 2θ angles, which comprises peaks at diffraction angles (2θ) as follows:

| 2θ (°) ± 0.2° | Intensity, % | 2θ (°)± 0.2° | Intensity, % | 2θ (°) ± 0.2° | Intensity, % |
|---|---|---|---|---|---|
| 7.65 | 21.23 | 20.30 | 56.69 | 26.47 | 100.00 |
| 10.76 | 60.50 | 20.50 | 29.39 | 27.48 | 10.36 |
| 13.12 | 49.64 | 20.73 | 31.87 | 28.03 | 12.42 |
| 14.56 | 74.92 | 20.98 | 40.35 | 30.20 | 15.04 |
| 14.68 | 53.96 | 21.36 | 85.73 | 31.16 | 53.82 |
| 15.39 | 23.44 | 22.48 | 15.11 | 32.06 | 17.19 |
| 16.15 | 28.42 | 23.25 | 14.22 | 32.85 | 3.74 |
| 16.96 | 44.87 | 23.52 | 16.46 | 34.76 | 3.14 |
| 19.50 | 6.17 | 24.69 | 6.27 | 35.20 | 8.08 |
| 19.86 | 11.48 | 25.27 | 7.16 | 37.35 | 4.44 |
| 20.10 | 41.65 | 26.02 | 5.47 | - | - |

In some embodiments, the crystalline form D of the compound of Formula (I) has an XRPD pattern as measured by using Cu-Ka radiation and expressed in 2θ angles, which comprises peaks at diffraction angles (2θ) as follows:

| 2θ (°) ± 0.2° | Interplanar distance, d(Å) | Intensity, % | 2θ (°) ± 0.2° | Interplanar distance, d(Å) | Intensity, % |
|---|---|---|---|---|---|
| 7.65 | 11.55 | 21.23 | 22.48 | 3.95 | 15.11 |
| 10.76 | 8.21 | 60.50 | 23.25 | 3.82 | 14.22 |
| 13.12 | 6.74 | 49.64 | 23.52 | 3.78 | 16.46 |
| 14.56 | 6.08 | 74.92 | 24.69 | 3.60 | 6.27 |
| 14.68 | 6.03 | 53.96 | 25.27 | 3.52 | 7.16 |
| 15.39 | 5.75 | 23.44 | 26.02 | 3.42 | 5.47 |
| 16.15 | 5.48 | 28.42 | 26.47 | 3.36 | 100.00 |
| 16.96 | 5.22 | 44.87 | 27.48 | 3.24 | 10.36 |
| 19.50 | 4.55 | 6.17 | 28.03 | 3.18 | 12.42 |
| 19.86 | 4.47 | 11.48 | 30.20 | 2.96 | 15.04 |
| 20.10 | 4.41 | 41.65 | 31.16 | 2.87 | 53.82 |
| 20.30 | 4.37 | 56.69 | 32.06 | 2.79 | 17.19 |
| 20.50 | 4.33 | 29.39 | 32.85 | 2.72 | 3.74 |
| 20.73 | 4.28 | 31.87 | 34.76 | 2.58 | 3.14 |
| 20.98 | 4.23 | 40.35 | 35.20 | 2.55 | 8.08 |
| 21.36 | 4.16 | 85.73 | 37.35 | 2.41 | 4.44 |

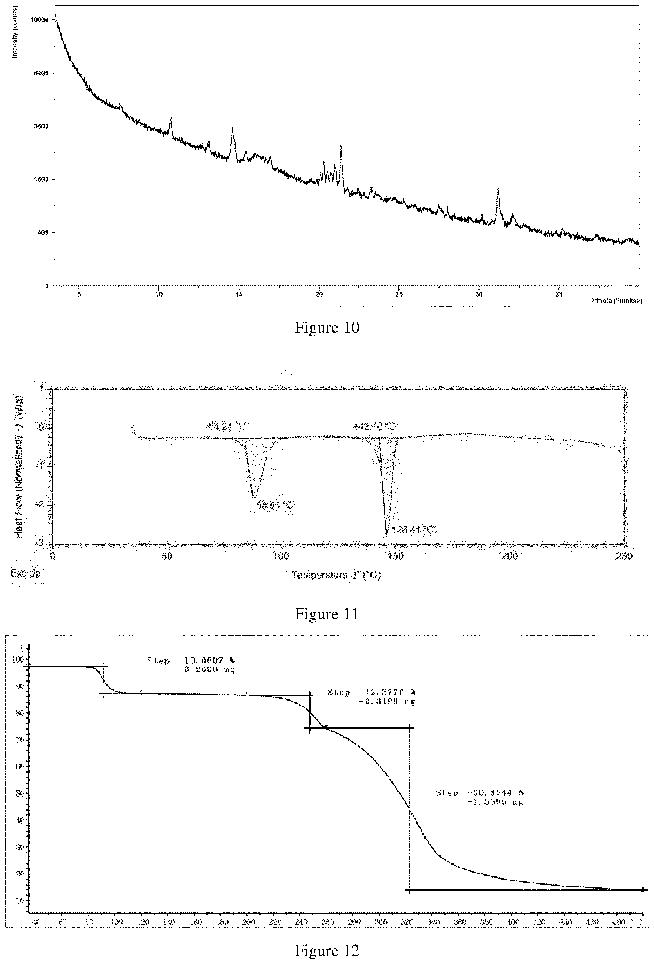

In some embodiments, the crystalline form D of the compound of Formula (I) has an XRPD pattern as measured by using Cu-Ka radiation and expressed in 2θ angles, which comprises peaks at the diffraction angles (2θ) substantially the same as those shown in Figure 10. In some embodiments, the crystalline form D of the compound of Formula (I) has XRPD peak positions as measured by using Cu-Ka radiation and expressed in 2θ angles, which are substantially the same as those shown in Figure 10.

In some embodiments, the crystalline form D of the compound of Formula (I) of the present application has a DSC curve which comprises characteristic peaks at about 84±5°C and 142±5°C (initial temperature). In some embodiments, the crystalline form D of the compound of Formula (I) has a DSC curve which comprises endothermic peaks in the range of about 75°C to 100°C and the range of 130°C to 155°C. In some embodiments, the crystalline form D of the compound of Formula (I) has a DSC curve which comprises characteristic peaks at substantially the same temperatures as shown in Figure 11. In some embodiments, the crystalline form D of the compound of Formula (I) has a DSC curve which comprises characteristic peak positions that are substantially the same as those shown in Figure 11.

In some embodiments, the crystalline form D of the compound of Formula (I) of the present application has a thermogravimetric analysis (TGA) curve which has a weight loss of 8% to 12% at about 70°C to 120°C, preferably has a weight loss of 9% to 11%, more preferably has a weight loss of 10.1%. In some embodiments, the crystalline form D of the compound of Formula (I) has a TGA curve which comprises the temperature substantially the same as that shown in Figure 12 at which a weight loss occurs. In some embodiments, the crystalline form D of the compound of Formula (I) has a TGA curve which is substantially the same as that shown in Figure 12.

In some embodiments, the crystalline form D of the compound of Formula (I) of the present application is an isopropyl acetate solvate, wherein the molar ratio of the compound of Formula (I) to the solvent is 1:0.5.

### Crystalline form E of the compound of Formula (I)

According to one embodiment of the present application, the present application provides a crystalline form E of the compound of Formula (I). The crystalline form E of the compound of Formula (I) has a XRPD pattern as measured by using Cu-Ka radiation and expressed in 2θ angles, which comprises characteristic peaks at the diffraction angles (2θ) of 10.8±0.2°, 14.7±0.2°, 15.8±0.2°, 16.6±0.2°, 17.2±0.2° and 23.8±0.2°. In some embodiments, the crystalline form E of the compound of Formula (I) has a XRPD pattern as measured by using Cu-Ka radiation and expressed in 2θ angles, which comprises characteristic peaks at the diffraction angles (2θ) of 10.8±0.2°, 13.1±0.2°, 14.7±0.2°, 15.0±0.2°, 15.6±0.2°, 15.8±0.2°, 16.6±0.2°, 17.2±0.2°, 19.2±0.2°, 22.7±0.2°, 23.5±0.2° and 23.8±0.2°. In some embodiments, the crystalline form E of the compound of Formula (I) has a XRPD pattern as measured by using Cu-Ka radiation and expressed in 2θ angles, which comprises characteristic peaks at the diffraction angles (2θ) of 7.8±0.2°, 10.8±0.2°, 12.2±0.2°, 12.7±0.2°, 13.1±0.2°, 14.7±0.2°, 15.0±0.2°, 15.6±0.2°, 15.8±0.2°, 16.6±0.2°, 17.2±0.2°, 19.2±0.2°, 19.9±0.2°, 20.1±0.2°, 21.0±0.2°, 21.7±0.2°, 22.7±0.2°, 23.5±0.2°, 23.8±0.2°, 27.6±0.2°, 27.8±0.2°, 30.1±0.2° and 32.0±0.2°. In some embodiments, the crystalline form E of the compound of Formula (I) has a XRPD pattern as measured by using Cu-Kα radiation and expressed in 2θ angles, which comprises peaks at the diffraction angles (2θ) as follows:

| 2θ (°) ± 0.2° | 2θ (°) ± 0.2° | 2θ (°) ± 0.2° |
|---|---|---|
| 7.83 | 20.41 | 27.82 |
| 10.80 | 20.97 | 28.64 |
| 12.28 | 21.25 | 29.47 |
| 12.70 | 21.73 | 30.11 |
| 13.11 | 22.14 | 30.24 |
| 14.67 | 22.71 | 30.80 |
| 14.96 | 23.50 | 31.20 |
| 15.65 | 23.80 | 31.99 |
| 15.79 | 24.10 | 32.86 |
| 16.63 | 24.66 | 33.72 |
| 17.18 | 25.19 | 34.74 |
| 18.55 | 25.86 | 35.81 |
| 19.16 | 27.26 | 37.45 |
| 19.88 | 27.60 | 39.06 |
| 20.09 | - | - |

In some embodiments, the crystalline form E of the compound of Formula (I) has an XRPD pattern as measured by using Cu-Ka radiation and expressed in 2θ angles, which comprises peaks at diffraction angles (2θ) as follows:

| 2θ (°)± 0.2° | Intensity, % | 2θ (°)± 0.2° | Intensity, % |
|---|---|---|---|
| 7.83 | 16.81 | 23.80 | 100.00 |
| 10.80 | 34.11 | 24.10 | 8.67 |
| 12.28 | 22.22 | 24.66 | 7.19 |
| 12.70 | 14.98 | 25.19 | 5.39 |
| 13.11 | 28.79 | 25.86 | 6.65 |
| 14.67 | 51.18 | 27.26 | 3.91 |
| 14.96 | 23.83 | 27.60 | 11.40 |
| 15.65 | 27.21 | 27.82 | 10.16 |
| 15.79 | 59.94 | 28.64 | 2.13 |
| 16.63 | 34.09 | 29.47 | 3.99 |
| 17.18 | 48.99 | 30.11 | 12.27 |
| 18.55 | 5.32 | 30.24 | 9.15 |
| 19.16 | 32.61 | 30.80 | 2.17 |
| 19.88 | 15.45 | 31.20 | 9.49 |
| 20.09 | 12.58 | 31.99 | 16.03 |
| 20.41 | 3.30 | 32.86 | 8.86 |
| 20.97 | 18.40 | 33.72 | 5.58 |
| 21.25 | 12.65 | 34.74 | 5.53 |
| 21.73 | 16.01 | 35.81 | 5.79 |
| 22.14 | 13.20 | 37.45 | 7.05 |
| 22.71 | 18.95 | 39.06 | 3.69 |
| 23.50 | 19.75 | - | - |

In some embodiments, the crystalline form E of the compound of Formula (I) has an XRPD pattern as measured by using Cu-Ka radiation and expressed in 2θ angles, which comprises peaks at diffraction angles (2θ) as follows:

| 2θ (°) ± 0.2° | Interplanar distance, d(Å) | Intensity, % | 2θ (°) ± 0.2° | Interplanar distance, d(Å) | Intensity, % |
|---|---|---|---|---|---|
| 7.83 | 11.28 | 16.81 | 23.80 | 3.74 | 100.00 |
| 10.80 | 8.18 | 34.11 | 24.10 | 3.69 | 8.67 |
| 12.28 | 7.20 | 22.22 | 24.66 | 3.61 | 7.19 |
| 12.70 | 6.97 | 14.98 | 25.19 | 3.53 | 5.39 |
| 13.11 | 6.75 | 28.79 | 25.86 | 3.44 | 6.65 |
| 14.67 | 6.03 | 51.18 | 27.26 | 3.27 | 3.91 |
| 14.96 | 5.92 | 23.83 | 27.60 | 3.23 | 11.40 |
| 15.65 | 5.66 | 27.21 | 27.82 | 3.20 | 10.16 |
| 15.79 | 5.61 | 59.94 | 28.64 | 3.11 | 2.13 |
| 16.63 | 5.33 | 34.09 | 29.47 | 3.03 | 3.99 |
| 17.18 | 5.16 | 48.99 | 30.11 | 2.97 | 12.27 |
| 18.55 | 4.78 | 5.32 | 30.24 | 2.95 | 9.15 |
| 19.16 | 4.63 | 32.61 | 30.80 | 2.90 | 2.17 |
| 19.88 | 4.46 | 15.45 | 31.20 | 2.86 | 9.49 |
| 20.09 | 4.42 | 12.58 | 31.99 | 2.80 | 16.03 |
| 20.41 | 4.35 | 3.30 | 32.86 | 2.72 | 8.86 |
| 20.97 | 4.23 | 18.40 | 33.72 | 2.66 | 5.58 |
| 21.25 | 4.18 | 12.65 | 34.74 | 2.58 | 5.53 |
| 21.73 | 4.09 | 16.01 | 35.81 | 2.51 | 5.79 |
| 22.14 | 4.01 | 13.20 | 37.45 | 2.40 | 7.05 |
| 22.71 | 3.91 | 18.95 | 39.06 | 2.30 | 3.69 |
| 23.50 | 3.78 | 19.75 | - | - | - |

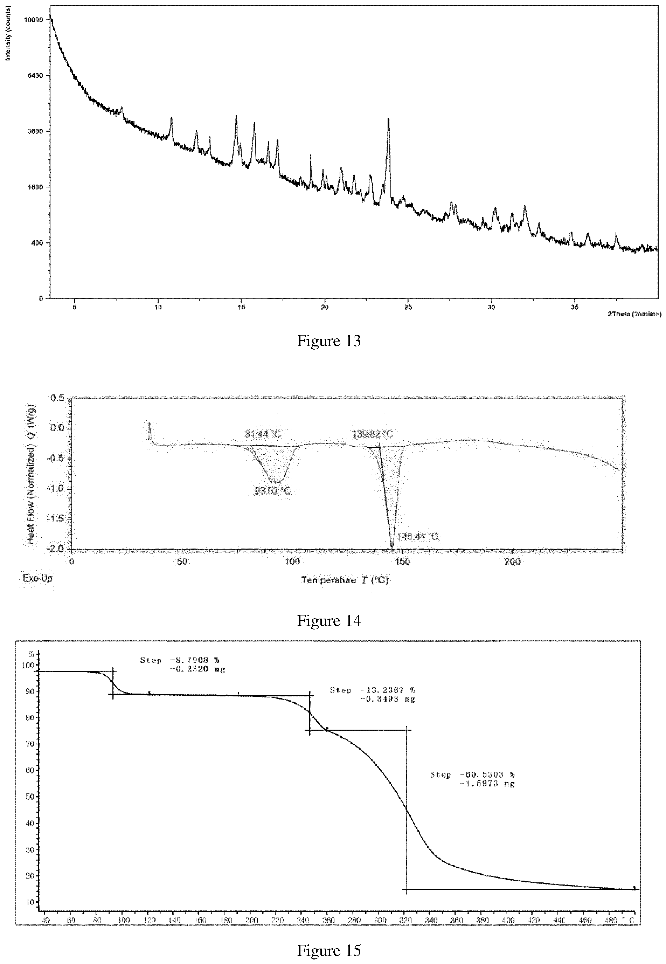

In some embodiments, the crystalline form E of the compound of Formula (I) has an XRPD pattern as measured by using Cu-Ka radiation and expressed in 2θ angles, which comprises peaks at the diffraction angles (2θ) substantially the same as those shown in Figure 13. In some embodiments, the crystalline form E of the compound of Formula (I) has XRPD peak positions as measured by using Cu-Ka radiation and expressed in 2θ angles, which are substantially the same as those shown in Figure 13.

In some embodiments, the crystalline form E of the compound of Formula (I) of the present application has a DSC curve which comprises characteristic peaks at about 81±5°C and 140±5°C (initial temperature). In some embodiments, the crystalline form E of the compound of Formula (I) has a DSC curve which comprises endothermic peaks in the range of about 75°C to 110°C and the range of 130°C to 155°C. In some embodiments, the crystalline form E of the compound of Formula (I) has a DSC curve which comprises characteristic peaks at substantially the same temperatures as shown in Figure 14. In some embodiments, the crystalline form E of the compound of Formula (I) has a DSC curve which comprises characteristic peak positions that are substantially the same as those shown in Figure 14.

In some embodiments, the crystalline form E of the compound of Formula (I) of the present application has a thermogravimetric analysis (TGA) curve which has a weight loss of 7% to 11% at about 70°C to 120°C, preferably has a weight loss of 8% to 10%, more preferably has a weight loss of 8.8%. In some embodiments, the crystalline form E of the compound of Formula (I) has a TGA curve which comprises the temperature substantially the same as that shown in Figure 15 at which a weight loss occurs. In some embodiments, the crystalline form E of the compound of Formula (I) has a TGA curve which is substantially the same as that shown in Figure 15.

In some embodiments, the crystalline form E of the compound of Formula (I) of the present application is a dimethyl carbonate solvate, wherein the molar ratio of the compound of Formula (I) to the solvent is 1:0.5.

### Amorphous form of the compound of Formula (I)

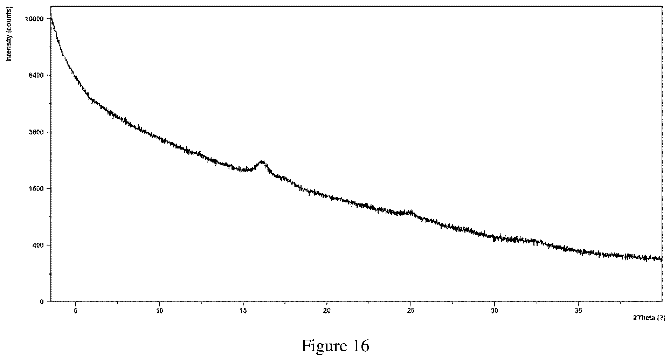

According to one embodiment of the present application, the present application provides an amorphous form of the compound of Formula (I), the amorphous form has an XRPD pattern as measured by using Cu-Ka radiation and expressed in 2θ angles, which does not have diffraction peaks. In some embodiments, the amorphous form has an XRPD pattern which is substantially the same as that shown in Figure 16.

According to some embodiments of the present application, a method of slow evaporation at room temperature is used to prepare crystalline forms, the method comprises dissolving the compound of Formula (I) in a solvent in a container to form a clear solution (if necessary, the solution can be filtered to obtain the clear solution; after filtering, the solvent is supplemented if necessary), sealing the container (e.g., using a sealing film), leaving a small hole or gap in the seal, and standing the clear solution to evaporate the solvent to obtain the crystalline forms. In some embodiments where the method of slow evaporation at room temperature is used to prepare crystalline forms, the solvent includes but is not limited to alcohols, ethers or esters, such as methanol, diethyl ether, ethyl acetate, isopropyl acetate, or dimethyl carbonate, or a mixed solvent formed with two or more of the above solvents. In some embodiments where the method of slow evaporation at room temperature is used to prepare crystalline forms, the weight-to-volume ratio (mg/mL) of the compound of Formula (I) to the solvent is (1 ~ 50) : 1, preferably (1 ~ 30) : 1 . In some embodiments, after a clear solution is obtained by filtration, a certain amount of the same type of solvent is further supplemented; preferably, the volume ratio of the supplemented solvent to the original solvent is 20 : 1-5. In some embodiments, the standing can be performed at room temperature.

In some embodiments of the present application, the solvents used in the preparation of crystalline forms by the method of slow evaporation at room temperature and results thereof in the present application are illustrated as follows:

| Solvent | Solid crystalline form |
|---|---|
| Methanol | Crystalline form A |
| Ethyl ether | Crystalline form B |
| Ethyl acetate | Crystalline form C |
| Isopropyl acetate | Crystalline form D |
| Dimethyl carbonate | Crystalline form E |

According to some embodiments of the present application, a method of slow cooling is used to prepare crystalline forms, the method comprises adding the compound of Formula (I) to a solvent, heating and stirring the resulting mixture to dissolve the compound of Formula (I), standing the resulting clear solution (if necessary, the solution can be filtered to obtain a clear solution), and slow cooling to obtain crystalline forms. In some embodiments where the method of slow cooling is used to prepare crystalline forms, the solvent includes but is not limited to aromatic hydrocarbons or ethers, or a mixed solvent of the above solvents, such as toluene or isopropyl ether. In some embodiments where the method of slow cooling is used to prepare crystalline forms, the slow cooling is performed at a cooling rate of 0.1 to 0.5°C/min, preferably 0.1 to 0.3°C/min, more preferably 0.1 °C/min. In some embodiments, the temperature to which the resulting mixture is heated is 50°C to 90°C, preferably 60°C to 80°C, such as 65°C, 70°C, or 75°C. In some embodiments, the temperature at the end of the cooling is room temperature or 0°C to 10°C, such as 3°C, 5°C, or 7°C. In some embodiments where the method of slow cooling is used to prepare crystalline forms, the weight-to-volume ratio (mg/mL) of the compound of Formula (I) to the solvent is (5 ~ 120):1, preferably (10 ~ 100):1. In some embodiments, the time of stirring is 10 to 50 minutes; preferably 30 minutes.

In some embodiments of the present application, the solvents used in the preparation of crystalline forms by the method of slow cooling and results thereof in the present application are illustrated as follows:

| Solvent | Solid crystalline form |
|---|---|
| Toluene | Crystalline form A |
| Isopropyl ether | Crystalline form A |

According to some embodiments of the present application, an method of anti-solvent addition is used to prepare crystalline forms, and the method comprises, but is not limited to, dissolving the compound of Formula (I) in a good solvent to form a clear solution (if necessary, the solution can be filtered to obtain the clear solution; the obtained clear solution may be heated if necessary), and then adding an anti-solvent to the clear solution, and stirring (the stirring can be performed at insulation, room temperature or cooling condition (e.g., cooling to 0°C to 20 °C, preferably 0°C to 10 °C, such as 0 °C, 5 °C or 10 °C)) to precipitate crystalline forms, or standing (e.g., standing at room temperature) (preferably slowly evaporating the solvents at the same time) to precipitate crystalline forms. In some embodiments where the method of anti-solvent addition is used to prepare crystalline forms, the good solvent includes but is not limited to alcohols, ketones, nitriles or ethers (including cyclic ethers or chain ethers), such as methanol, ethanol, isopropanol, n-propanol, n-butanol, acetone, butanone, acetonitrile, or tetrahydrofuran, etc. In some embodiments where the method of anti-solvent addition is used to prepare crystalline forms, the anti-solvent includes but is not limited to inorganic solvents, such as water. In some embodiments where the method of anti-solvent addition is used to prepare crystalline forms, the volume ratio of the good solvent to the anti-solvent is 4: (1 ~ 5), preferably 4:3. In some embodiments, the weight-to-volume ratio (mg/mL) of the compound of Formula (I) to the good solvent is (120 ~ 80) : 1, preferably 100:1.

In some embodiments of the present application, the solvents used in the preparation of crystalline forms by the method of anti-solvent addition and results thereof in the present application are illustrated as follows:

| Solvent | | Solid crystalline form |
|---|---|---|
| Good solvent | Anti-solvent | |
| Methanol | Water | Crystalline form A |
| Ethanol | | |
| Isopropanol | | |
| n-propanol | | |
| n-butanol | | |
| Acetone | | |
| butanone | | |
| Acetonitrile | | |
| Tetrahydrofuran | | |

According to some embodiments of the present application, a method of suspending and stirring at room temperature is used to prepare crystalline forms, and the method comprises, but is not limited to, adding a solid form (e.g., amorphous form) of the compound of Formula (I) to a solvent to obtain a suspension, stirring at room temperature, and then separating to obtain crystalline forms. In some embodiments where the method of suspending and stirring at room temperature is used to prepare crystalline forms, the solvent includes but is not limited to inorganic solvents (e.g., water) and organic solvents (e.g., alkanes, ethers or aromatic hydrocarbons), such as water, n-hexane, n-heptane, cyclohexane, n-pentane, isopropyl ether, or toluene, or a mixed solvent of two or more selected from the above solvents. In some embodiments where the method of suspending and stirring at room temperature is used to prepare crystalline forms, the weight-to-volume ratio (mg/mL) of the compound of Formula (I) to the solvent is (10 ~ 50) : 1, preferably (20 ~ 40) : 1 .

In some embodiments of the present application, the solvents used in the preparation of crystalline forms by the method of suspending and stirring at room temperature and results thereof in the present application are illustrated as follows:

| Solvent | Solid crystalline form |
|---|---|
| Water | Crystalline form A |
| n-Hexane | |
| n-Heptane | |
| Cvclohexane | |
| n-Pentane | |
| Isopropyl ether | |
| Toluene | |

According to some embodiments of the present application, an method of anti-solvent addition is used to prepare the amorphous form, and the method comprises, but is not limited to, dissolving the compound of Formula (I) in a good solvent to form a clear solution, and then adding an anti-solvent to the clear solution, thereby precipitating the amorphous form. In some embodiments where the method of anti-solvent addition is used to prepare the amorphous form, the good solvent includes but is not limited to esters and ethers, such as ethyl acetate, methyl tert-butyl ether, isopropyl ether, and the like. In some embodiments where the method of anti-solvent addition is used to prepare the amorphous form, the anti-solvent includes but is not limited to hydrocarbons, such as n-heptane.

According to some embodiments of the present application, a method of reduced-pressure concentration is used to prepare the amorphous form, and the method comprises, but is not limited to, dissolving the compound of Formula (I) in a good solvent to form a clear solution, and then concentrating the solution under reduced pressure to precipitate the amorphous form. In some embodiments where the method of reduced-pressure concentration is used to prepare the amorphous form, the good solvent includes but is not limited to alcohols, such as methanol and the like. In some embodiments where the method of reduced-pressure concentration is used to prepare the amorphous form, the weight-to-volume ratio (mg/mL) of the compound of Formula (I) to the solvent is (10 ~ 50) : 1, preferably (20 ~ 40) : 1 .

The publication WO2019105234A1 of the international patent application No. PCT/CN2018/115615 discloses the method for preparing the compound of Formula (I) of the present application, such as Example 5, and also discloses the therapeutic activity of the compound, see Pharmacological Tests, including Test Examples 1 to 4. In the present application, the full text of WO2019105234A1 is incorporated by reference.

The crystalline form A, B, C, D or E of the compound of Formula (I), or the amorphous form of the compound of Formula (I) according to the present application, has obvious agonistic effect on PPAR δ, and low toxicity to HepG2 cells and HEK293 cells, no obvious inhibitory effect on hERG, and has no potential risks that may lead to prolonged cardiac QT interval.

### Pharmaceutical composition and use

Another object of the present application is to provide a pharmaceutical composition, which comprises:
i) a solid form of the compound of Formula (I) of the present application, more particularly the crystalline form A, B, C, D or E of the compound of Formula (I), or the amorphous form of the compound of Formula (I), or any combination thereof; and
ii) one or more pharmaceutically acceptable carriers.

Another object of the present application is to provide a method for treating a disease associated with peroxisome proliferator-activated receptor (PPAR) such as non-alcoholic fatty liver disease (NAFLD) in an individual, which comprises administering to an individual in need thereof a therapeutically effective amount of the crystalline form A, B, C, D or E of the compound of Formula (I), or the amorphous form of the compound of Formula (I), or any combination thereof according to the present application.

Another object of the present application is to provide the crystalline form A, B, C, D, or E of the compound of Formula (I), or the amorphous form of the compound of Formula (I), or any combination thereof according to the present application, for use in the treatment of a disease associated with peroxisome proliferator-activated receptor (PPAR) such as non-alcoholic fatty liver disease (NAFLD) in an individual.

Another object of the present application is to provide use of the crystalline form A, B, C, D or E of the compound of Formula (I), or the amorphous form of the compound of Formula (I), or any combination thereof according to the present application in the manufacture of a medicament for the treatment of a disease associated with peroxisome proliferator-activated receptor (PPAR) such as non-alcoholic fatty liver disease (NAFLD) in an individual.

According to some embodiments of the present application, the disease associated with peroxisome proliferator-activated receptor (PPAR) such as non-alcoholic fatty liver disease (NAFLD) described in the present application includes non-alcoholic fatty liver disease (NAFLD).

The term "pharmaceutically acceptable carrier" as used herein refers to a diluent, adjuvant, excipient or vehicle administered together with a therapeutic agent, and which is suitable for contacting tissues of human and/or other animal without excessive toxicity, irritation, allergic reactions, or without other problems or complications corresponding to a reasonable benefit/risk ratio within the scope of reasonable medical judgment.

The pharmaceutically acceptable carriers that can be used in the pharmaceutical composition of the present application include, but are not limited to, sterile liquids, such as water and oils, including those oils derived from petroleum, animals, vegetables or synthetic origins, for example, peanut oil, soybean oil, mineral oil, sesame oil, etc. When the pharmaceutical composition is administered intravenously, water is an exemplary carrier. It is also possible to use physiological saline and glucose in aqueous solution and glycerol in aqueous solution as liquid carriers, especially for injections. Suitable pharmaceutical excipients include starch, glucose, lactose, sucrose, gelatin, maltose, chalk, silica gel, sodium stearate, glyceryl monostearate, talc, sodium chloride, skimmed milk powder, glycerin, propylene glycol, water, ethanol, etc. The composition can also comprise a small amount of wetting agent, emulsifier or pH buffering agent as needed. Oral preparations can comprise standard carriers, such as pharmaceutical grade mannitol, lactose, starch, magnesium stearate, sodium saccharin, cellulose, magnesium carbonate and the like. Examples of suitable pharmaceutically acceptable carriers are described in Remington's Pharmaceutical Sciences (1990).

The pharmaceutical composition of the present application can take effect systemically and/or locally. For this purpose, it can be administered by a suitable route, such as by injection, intravenous, intraarterial, subcutaneous, intraperitoneal, intramuscular or transdermal administration; or by oral, buccal, transnasal, transmucosal, topical administration, or be administered in the form of ophthalmic preparations or by inhalation.

For these administration routes, the composition of the present application can be administered in a suitable preparation form.

The preparation form can be a solid preparation, a semi-solid preparation, a liquid preparation or a gaseous preparation, including but not limited to tablets, capsules, powders, granules, lozenges, hard candy, powders, sprays, creams, ointments, suppositories, gels, pastes, lotions, ointments, aqueous suspensions, injectable solutions, suspensions, elixirs, syrups.

The pharmaceutical composition described in the present application can be prepared by any method well known in the art, for example, by mixing, dissolving, granulating, sugar coating, milling, emulsifying, freeze-drying and other treatments.

The term "therapeutically effective amount" as used herein refers to an amount of the compound that will alleviate one or more symptoms of a condition to be treated to a certain extent after being administered.

The dosage regimen can be adjusted to provide the best desired response. For example, a single bolus can be administered, several divided doses can be administered over time, or the dose can be proportionally reduced or increased as indicated by the urgent need for the treatment situation. It should be noted that the dose value can vary with the type and severity of the disease or condition to be alleviated, and can include single or multiple doses. It should be further understood that for any one particular individual, the specific dosage regimen should be adjusted over time according to the needs of the individual and the professional judgment of the person administering the composition or supervising the administration of the composition.

The amount of the compound of the present application administered will depend on the individual to be treated, the severity of disease or condition, the rate of administration, the treatment of the compound, and the judgment of the prescription physician. Generally, the effective dose is about 0.0001 to about 50 mg per kg of body weight per day, for example, about 0.01 to about 10 mg/kg/day (single or divided administration). For a 70 kg person, this would add up to about 0.007 mg/day to about 3500 mg/day, for example, about 0.7 mg/day to about 700 mg/day. In some cases, a dose level not higher than the lower limit of the aforementioned range may be sufficient, while in other cases, a larger dose can still be used without causing any harmful side effects, provided that the larger dose is divided into several smaller doses to be administered throughout whole day.

The content or amount of the compound of the present application in the pharmaceutical composition can be about 0.01 mg to about 1000 mg, suitably 0.1 to 500 mg, preferably 0.5 to 300 mg, more preferably 1 to 150 mg, particularly preferably 1 to 50 mg, such as 1.5 mg, 2 mg, 4 mg, 10 mg and 25 mg.

Unless otherwise specified, as used herein, the term "treating" refers to reversing, alleviating, inhibiting the progress of one or more symptoms of a disorder or condition to which such term is applied, or preventing such disorder or condition or one or more symptoms of such disorder or condition.

The term "individual" as used herein includes human or non-human animals. Exemplary human individuals include human individuals (referred to as patients) or normal individuals suffering from diseases such as those described herein. In the present application, "non-human animals" include all vertebrates, such as non-mammals (such as birds, amphibians, reptiles) and mammals, such as non-human primates, domestic animals, and/or domesticated animals (such as sheep, dogs, cats, cows, pigs, etc.).

### Example

The present application will be explained in more detail below in combination with the examples. The examples of the present application are only used to illustrate the technical solutions of the present application and are not used to limit the scope of the present application. Those skilled in the art can make some non-essential improvements and adjustments, which still belong to the protection scope of the present application.

Test equipment information and methods used in the experiment:
Method 1: X-ray powder diffraction (XRPD)
   X'Pert3 Powder type powder diffractometer was used. The instrument was irradiated with Cu target, Cu Kα1=1.5406A, monochromatic radiation, voltage: 40 kV, current: 40 mA excitation, and Absolute scan was used for detection at room temperature. The detection range was from 3.5° to 40°, the step length was 0.013°, the dwell time was 50s, and the scan was performed once.
Method 2: Differential scanning calorimetry analysis (DSC)
   Equipment information: DSC2500(TA)
   Test method: thermal analysis method (Chinese Pharmacopoeia, 2015 Edition, Four General Rules 0661)
   Test conditions: heating rate: 10°C/min, starting temperature: 35.0°C
   Sample preparation: about 2~5mg of sample to be tested was weighed, spread in a 60µl aluminum crucible with hole, and an empty aluminum crucible was used as a reference, then the sample was loaded and measured.
Method 3: Thermogravimetric analysis (TGA)
   Equipment information: Mettler-Toledo TGA
   Test method: thermal analysis method (Chinese Pharmacopoeia, 2015 Edition, Four General Rules 0661)
   Test conditions: starting temperature: 35.0 °C, ending temperature: 500.0 °C, heating rate: 10°C/min

Method of for preparing the compound sample used in the experiments:
The sample of the compound of Formula (I) was prepared according to the method described in Example 5 in the international patent application No. PCT/CN2018/115615.

The sample of amorphous form of the compound of Formula (I) used in Examples 6 and 7 was prepared according to the method described in Example 12.

### Example 1

50 mg of a sample of the compound of Formula (I) was weighed and dissolved in 4 mL of methanol until clear, evaporated at room temperature to remove the solvent, and the solid was precipitated and collected. The solid was detected by XRPD, and the obtained XRPD pattern was shown in Figure 1. The obtained solid was the crystalline form A of the compound of Formula (I) of the present application, which was subjected to differential scanning calorimetry analysis (DSC) and the obtained DSC curve was shown in Figure 2, and which was subjected to thermogravimetric analysis (TGA) and the obtained TGA curve was shown in Figure 3.

### Example 2.

200 mg of a sample of the compound of Formula (I) was weighed and added in 2.0 mL of toluene, stirred and heated to 70°C for dissolution until clear, stirred for 30 min when the temperature was kept, allowed to stand and to be cooled to room temperature, and the solid was precipitated and collected. The obtained solid was subjected to XRPD detection, and the result showed that the obtained product was the same as the crystalline form A obtained in Example 1.

### Example 3.

200 mg of a sample of the compound of Formula (I) was weighed and added in 17 mL of isopropyl ether, stirred and heated to 70°C for dissolution until clear, stirred for 30 min when the temperature was kept, allowed to stand and to be cooled to room temperature, and the solid was precipitated and collected. The obtained solid was subjected to XRPD detection, and the result showed that the obtained product was the same as the crystalline form A obtained in Example 1.

### Example 4.

2.0g of a sample of the compound of Formula (I) was weighed, added with 20mL of ethanol at room temperature until it was just dissolved and clear, heated to 50°C, 15mL of water was added dropwise when the temperature was kept. After the addition was completed, the resulting mixture was stirred for 2h when the temperature was kept, then stirred and cooled to room temperature, stirred for 16h when the temperature was kept, and then filtered and dried under vacuum at 50°C for 6h, a solid was collected. The obtained solid was subjected to XRPD detection, and the result showed that the obtained product was the same as the crystalline form A obtained in Example 1.

### Example 5.

Several 50 mg of samples of the compound of Formula (I) were weighed, and placed into different glass bottles, respectively. They were respectively dissolved with any of the good solvents in the following table until clear, then water was slowly added dropwise until turbid. The resulting mixture was heated and dissolved until clear and supplemented with water, heated and dissolved until clear, and then cooled down to room temperature, and allowed to stand until crystals precipitated.

| Solvent | | Solid crystalline form |
|---|---|---|
| Good-solvent | Anti-solvent | |
| Methanol | Water | Crystalline form A |
| Ethanol | | Crystalline form A |
| Isopropanol | | Crystalline form A |
| n-Propanol | | Crystalline form A |
| n-Butanol | | Crystalline form A |
| Acetone | | Crystalline form A |
| Butanone | | Crystalline form A |
| Acetonitrile | | Crystalline form A |
| Tetrahydrofuran | | Crystalline form A |

### Example 6.

30 mg of the amorphous sample of the compound of Formula (I) was weighed and added in 1.0 mL of water, suspended and stirred for 48 hours, filtered, and the resultant solid was collected. The obtained solid was subjected to XRPD detection, and the result showed that the obtained product was the same as the crystalline form A obtained in Example 1.

### Example 7.

A method similar to that of Example 6 was adopted to prepare crystalline forms of the compound of Formula (I), except that the water in Example 6 was replaced with n-hexane, n-heptane, cyclohexane, n-pentane, isopropyl ether, or toluene, and the obtained solid was subjected to XRPD. The result showed that the obtained products were the same as the crystalline form A obtained in Example 1.

### Example 8.

50 mg of a sample of the compound of Formula (I) was weighed, added in 2.0 mL of ethyl ether and dissolved until clear, and then filtered, the resulting filtrate was collected and supplemented with 300 µL of ethyl ether. The ethyl ether was evaporated at room temperature to precipitate a solid, and the solid was collected. The obtained solid was subjected to XRPD detection, and the result was shown in Figure 4. The obtained solid was the crystalline form B of the compound of Formula (I) of the present application, which was subjected to differential scanning calorimetry analysis (DSC) and the obtained DSC curve was shown in Figure 5, and which was subjected to thermogravimetric analysis (TGA) and the obtained TGA curve was shown in Figure 6.

### Example 9.

50 mg of a sample of the compound of Formula (I) was weighed, added in 2.0 mL of ethyl acetate, dissolved until clear, and filtered, the resulting filtrate was collected and supplemented with 300 µL of ethyl acetate. The ethyl acetate was evaporated at room temperature to precipitate a solid, and the solid was collected. The obtained solid was subjected to XRPD detection, and the result was shown in Figure 7. The obtained solid was the crystalline form C of the compound of Formula (I) of the present application, which was subjected to differential scanning calorimetry analysis (DSC) and the obtained DSC curve was shown in Figure 8, and which was subjected to thermogravimetric analysis (TGA) and the obtained TGA curve was shown in Figure 9.

### Example 10.

50 mg of a sample of the compound of Formula (I) was weighed, added in 2.0 mL of isopropyl acetate, dissolved until clear, and filtered, the filtrate was collected and supplemented with 300 µL of isopropyl acetate. The isopropyl acetate was evaporated at room temperature to precipitate a solid, and the solid was collected. The obtained solid was subjected to XRPD detection, and the result was shown in Figure 10. The obtained solid was the crystalline form D of the compound of Formula (I) of the present application, which was subjected to differential scanning calorimetry analysis (DSC) and the obtained DSC curve was shown in Figure 11, and which was subjected to thermogravimetric analysis (TGA) and the obtained TGA curve was shown in Figure 12.

### Example 11.

50 mg of a sample of the compound of Formula (I) was weighed, added in 2.0 mL of dimethyl carbonate, dissolved until clear, and filter, the filtrate was collected and supplemented with 300 µL of dimethyl carbonate. The dimethyl carbonate was evaporated at room temperature to precipitate a solid, and the solid was collected. The obtained solid was subjected to XRPD detection, and the result was shown in Figure 13. The obtained solid was the crystalline form E of the compound of Formula (I) of the present application, which was subjected to differential scanning calorimetry analysis (DSC) and the obtained DSC curve was shown in Figure 14, and which was subjected to thermogravimetric analysis (TGA) and the obtained TGA curve was shown in Figure 15.

### Example 12.

A sample of 200 mg of the compound of Formula (I) was weighed, dissolved in 20 ml of methanol until clear, and concentrated under reduced pressure to remove the solvent. The solid was precipitated and collected. The obtained solid was subjected to XRPD detection, and the obtained XRPD pattern was shown in Figure 16, which showed that it was amorphous.

### Example 13.

30 mg of a sample of the compound of Formula (I) was weighed, added into ethyl acetate at room temperature and dissolved until clear, n-heptane was slowly added until a large amount of solid precipitated, and the solid was filtered and collected. The obtained solid was detected by XRPD, and its XRPD pattern did not have diffraction peaks, indicating that the obtained product was amorphous.

### Example 14.

30 mg of a sample of the compound of Formula (I) was weighed, added into methyl tert-butyl ether at room temperature and dissolved until clear, n-heptane was slowly added until a large amount of solid precipitated, and the solid was filtered and collected. The obtained solid was detected by XRPD, and its XRPD pattern did not have diffraction peaks, indicating that the obtained product was amorphous.

### Example 15.

30 mg of a sample of the compound of Formula (I) was weighed, added into isopropyl ether at room temperature and dissolved until clear, n-heptane was slowly added until a large amount of solid precipitated, and the solid was filtered and collected. The obtained solid was detected by XRPD, and its XRPD pattern did not have diffraction peaks, indicating that the obtained product was amorphous.

### Experimental Example 1: Study on pharmacokinetics (PK) in rats

A sample of crystalline form A was administered to male SD rats by intragastric route (PO) to investigate its pharmacokinetic characteristics. The dose was 5 mg/kg, and 0.5% MC (methylcellulose sodium) was used as a solvent to prepare a suspension of crystalline form A. In the administration group, whole blood samples were collected before PO administration (0h) and at time points of 0.083, 0.17, 0.25, 0.5, 1, 2, 4, 6, 8 and 24 hours after administration. The whole blood samples were anticoagulated with EDTA.K₂, and plasma samples were obtained after centrifugation and stored at -80°C for later testing. In this experiment, there were three animals in each group for parallel testing.

The plasma samples were treated by precipitating protein and then analyzed by LC-MS/MS. Using WinNonlin 6.3 software, the pharmacokinetic parameters was calculated by means of the non-compartmental model, and the results were shown in Table 1.

**Table 1: Pharmacokinetic parameters of compound crystalline form A administered by PO in rat blood**

| Route of administration | Dose | AUCₗₐₛₜ | Cmax |
|---|---|---|---|
| | mg/kg | h^{∗}ng/mL | ng/mL |
| PO | 5 | 777 | 522 |

As shown in Table 1, the crystalline form A administered by PO at a dose of 5 mg/kg had a better exposure in rat plasma.

### Experimental Example 2: Stability test

In this experimental example, the chemical stability of crystalline form A was studied by high temperature and high humidity test.

Method for testing purity: high performance liquid chromatography (Chinese Pharmacopoeia, 2015 Edition, Four General Rules 0512) was used to test purify.
Chromatographic column: phenyl bonded silica gel was used as filler;
Mobile phase A: 5mM ammonium acetate (pH 4.0);
Mobile phase B: Acetonitrile (containing 0.05% acetic acid);
Detection wavelength: 230nm.
Elution condition: gradient elution.
Experimental Example 2-1: High temperature stability test

The crystalline form A was placed in a sealed and clean glass bottle, and then the glass bottle was placed in a 60°C constant temperature drying oven, and samples were taken on the 11^{th} day and the 30^{th} day, respectively, to detect impurity contents, and the purity results were shown in Table 2.

**Table 2: High temperature stability data of crystalline form A**

| Time | Purity/% |
|---|---|
| 0^{th} day | 99.51 |
| 11^{th} day | 99.53 |
| 30^{th} day | 99.54 |

It could be seen from Table 2 that the purity of crystalline form A showed no significant change under high temperature conditions. The XRPD testing showed that the crystalline form did not change after 30 days of storage, indicating that the crystalline form A had high temperature resistance.

### Experimental Example 2-2: High humidity stability test

The crystalline form A of the compound of Formula I was evenly spread into an open culture dish with a thickness of ≤5mm, and placed in a constant temperature incubator at room temperature (25±2°C) and relative humidity of 92.5% RH. Samples were taken on the 11^{th} day and the 30^{th} day, respectively, to detect impurity contents, and the purity results were shown in Table 3.

**Table 3: High humidity stability data of crystalline form A**

| Time | Purity/% |
|---|---|
| 0^{th} day | 99.51 |
| 10^{th} day | 99.53 |
| 30^{th} day | 99.54 |

It can be seen from Table 3 that the purity of crystalline form A had no significant change under high humidity conditions, and the XRPD testing showed that the crystalline form did not change after 30 days of storage, that was, it had high humidity resistance.

The above specific embodiments further describe the present application in detail. However, it should be understood that the scope of the above-mentioned subject matter of the present application is not limited to the listed embodiments, and all technical solutions implemented based on the content of the present application fall within the scope of the present application.

## Claims

1. A crystalline form A of the compound of Formula (I), 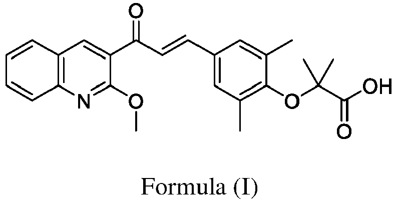
wherein the crystalline form A of the compound of Formula (I) has an XRPD pattern as measured by using Cu-Ka radiation and expressed in 2θ angles, comprising characteristic peaks at diffraction angles (2θ) of 11.1±0.2°, 11.5±0.2°, 16.2±0.2°, 17.0±0.2°, 19.0±0.2° and 24.9±0.2°;
preferably comprising characteristic peaks at diffraction angles (2θ) of 7.3±0.2°, 11.1±0.2°, 11.5±0.2°, 16.0±0.2°, 16.2±0.2°, 16.9±0.2°, 19.0±0.2°, 20.9±0.2° and 24.9±0.2°;
preferably comprising characteristic peaks at diffraction angles (2θ) of 7.3±0.2°, 8.2±0.2°, 11.1±0.2°, 11.5±0.2°, 11.9±0.2°, 16.0±0.2°, 16.2±0.2°, 16.9±0.2°, 19.0±0.2°, 20.9±0.2°, 24.0±0.2° and 24.9±0.2°;
preferably, the crystalline form A of the compound of Formula (I) has an XRPD pattern which is substantially the same as that shown in Figure 1;
preferably, the crystalline form A of the compound of Formula (I) has a DSC curve comprising an endothermic peak in the range of about 130°C to 160°C.

2. A method for preparing the crystalline form A of the compound of Formula (I) according to claim 1, wherein the method is selected from the group consisting of a method of anti-solvent addition, a method of slow evaporation at room temperature, a method of suspending and stirring at room temperature, and a method of slow cooling.

3. A crystalline form B of the compound of Formula (I), 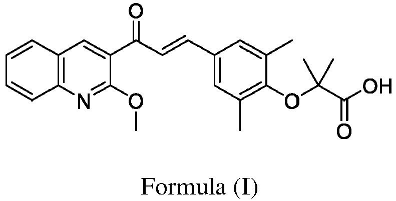
wherein the crystalline form B of the compound of Formula (I) has an XRPD pattern as measured by using Cu-Ka radiation and expressed in 2θ angles, comprising characteristic peaks at diffraction angles (2θ) of 7.5±0.2°, 10.8±0.2°, 12.4±0.2°, 14.7±0.2°, 18.2±0.2° and 25.3±0.2°;
preferably comprising characteristic peaks at diffraction angles (2θ) of 7.5±0.2°, 10.8±0.2°, 12.4±0.2°, 14.7±0.2°, 14.8±0.2°, 15.0±0.2°, 16.4±0.2°, 17.1±0.2°, 18.2±0.2°, 20.9±0.2°, 21.2±0.2°, 23.4±0.2° and 25.3±0.2°;
preferably comprising characteristic peaks at diffraction angles (2θ) of 7.5±0.2°, 10.8±0.2°, 12.4±0.2°, 14.7±0.2°, 14.8±0.2°, 15.0±0.2°, 16.4±0.2°, 17.1±0.2°, 18.2±0.2°, 19.2±0.2°, 20.1±0.2°, 20.9±0.2°, 21.2±0.2°, 23.4±0.2°, 25.3±0.2°, 28.5±0.2°, 30.3±0.2°, 33.8±0.2°, 35.8±0.2°, and 36.8±0.2°;
preferably, the crystalline form B of the compound of Formula (I) has an XRPD pattern which is substantially the same as that shown in Figure 4.

4. A method for preparing the crystalline form B of the compound of Formula (I) according to claim 3, wherein the method is a method of slow evaporation at room temperature.

5. A crystalline form C of the compound of Formula (I): 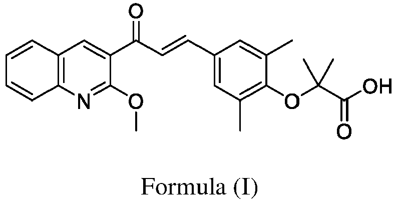
wherein the crystalline form C of the compound of Formula (I) has a XRPD pattern as measured by using Cu-Ka radiation and expressed in 2θ angles, comprising characteristic peaks at the diffraction angles (2θ) of 7.8±0.2°, 14.6±0.2°, 17.1±0.2°, 21.6±0.2° and 22.7±0.2°;
preferably comprising characteristic peaks at the diffraction angles (2θ) of 7.8±0.2°, 14.6±0.2°, 15.6±0.2°, 17.1±0.2°, 20.4±0.2°, 20.6±0.2°, 21.6±0.2°, 22.7±0.2°, 29.3±0.2°, 29.6±0.2° and 31.3±0.2°;
preferably comprising characteristic peaks at the diffraction angles (2θ) of 7.8±0.2°, 9.6±0.2°, 10.8±0.2°, 12.7±0.2°, 13.1±0.2°, 14.6±0.2°, 15.6±0.2°, 17.1±0.2°, 18.6±0.2°, 20.4±0.2°, 20.6±0.2°, 21.1±0.2°, 21.6±0.2°, 22.3±0.2°, 22.7±0.2°, 23.6±0.2°, 27.2±0.2°, 27.8±0.2°, 28.7±0.2°, 29.3±0.2°, 29.6±0.2° and 31.3±0.2°;
preferably, the crystalline form C of the compound of Formula (I) has an XRPD pattern which is substantially the same as that shown in Figure 7.

6. A method for preparing the crystalline form C of the compound of Formula (I) according to claim 5, wherein the method is a method of slow evaporation at room temperature.

7. A crystalline form D of the compound of Formula (I): 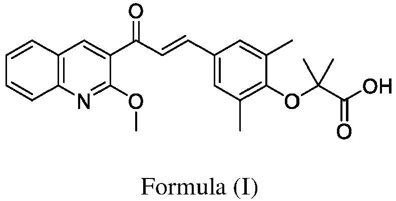
wherein the crystalline form D of the compound of Formula (I) has a XRPD pattern as measured by using Cu-Ka radiation and expressed in 2θ angles, comprising characteristic peaks at the diffraction angles (2θ) of 10.8±0.2°, 14.6±0.2°, 14.7±0.2°, 20.3±0.2°, 21.4±0.2°, 26.5±0.2° and 31.2±0.2°;
preferably comprising characteristic peaks at the diffraction angles (2θ) of 10.8±0.2°, 13.1±0.2°, 14.6±0.2°, 14.7±0.2°, 17.0±0.2°, 20.1±0.2°, 20.3±0.2°, 20.5±0.2°, 20.7±0.2°, 21.0±0.2°, 21.4±0.2°, 26.5±0.2° and 31.2±0.2°;
preferably comprising characteristic peaks at the diffraction angles (2θ) of 7.6±0.2°, 10.8±0.2°, 13.1±0.2°, 14.6±0.2°, 14.7±0.2°, 15.4±0.2°, 16.1±0.2°, 17.0±0.2°, 19.9±0.2°, 20.1±0.2°, 20.3±0.2°, 20.5±0.2°, 20.7±0.2°, 21.0±0.2°, 21.4±0.2°, 22.5±0.2°, 23.3±0.2°, 23.5±0.2°, 26.5±0.2°, 30.2±0.2°, 31.2±0.2° and 32.1±0.2°;
preferably, the crystalline form D of the compound of Formula (I) has an XRPD pattern which is substantially the same as that shown in Figure 10.

8. A method for preparing the crystalline form D of the compound of Formula (I) according to claim 7, wherein the method is a method of slow evaporation at room temperature.

9. A crystalline form E of the compound of Formula (I): 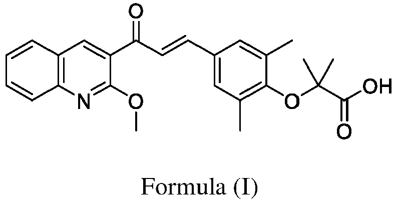
wherein the crystalline form E of the compound of Formula (I) has a XRPD pattern as measured by using Cu-Ka radiation and expressed in 2θ angles, comprising characteristic peaks at the diffraction angles (2θ) of 10.8±0.2°, 14.7±0.2°, 15.8±0.2°, 16.6±0.2°, 17.2±0.2° and 23.8±0.2°;
preferably comprising characteristic peaks at the diffraction angles (2θ) of 10.8±0.2°, 13.1±0.2°, 14.7±0.2°, 15.0±0.2°, 15.6±0.2°, 15.8±0.2°, 16.6±0.2°, 17.2±0.2°, 19.2±0.2°, 22.7±0.2°, 23.5±0.2° and 23.8±0.2°;
preferably comprising characteristic peaks at the diffraction angles (2θ) of 7.8±0.2°, 10.8±0.2°, 12.2±0.2°, 12.7±0.2°, 13.1±0.2°, 14.7±0.2°, 15.0±0.2°, 15.6±0.2°, 15.8±0.2°, 16.6±0.2°, 17.2±0.2°, 19.2±0.2°, 19.9±0.2°, 20.1±0.2°, 21.0±0.2°, 21.7±0.2°, 22.7±0.2°, 23.5±0.2°, 23.8±0.2°, 27.6±0.2°, 27.8±0.2°, 30.1±0.2° and 32.0±0.2°;
preferably, the crystalline form E of the compound of Formula (I) has XRPD pattern which is substantially the same as that shown in Figure 13.

10. A method for preparing the crystalline form E of the compound of Formula (I) according to claim 9, wherein the method is a method of slow evaporation at room temperature.

11. An amorphous form of the compound of Formula (I): 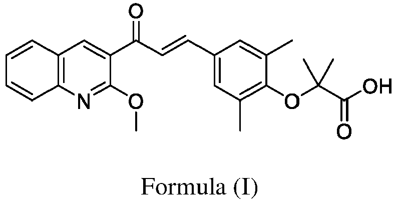 wherein the amorphous form has an XRPD pattern as measured by using Cu-Ka radiation and expressed in 2θ angles, which does not have diffraction peaks.

12. A method for preparing the amorphous form of the compound of Formula (I) according to claim 11, wherein the method is selected from the group consisting of a method of anti-solvent addition and a method of slow evaporation at room temperature.

13. A pharmaceutical composition comprising the crystalline form A, B, C, D, E or amorphous form of the compound of Formula (I) according to any one of claims 1, 3, 5, 7, 9 and 11, and one or more pharmaceutically acceptable carriers.

14. Use of the crystalline A, B, C, D, E or amorphous form of the compound of Formula (I) according to any one of claims 1, 3, 5, 7, 9 and 11 in the manufacture of a medicament for prevention or treatment of a disease associated with peroxisome proliferator-activated receptor (PPAR), such as non-alcoholic fatty liver disease (NAFLD).

15. A method for treating a disease associated with peroxisome proliferator-activated receptor (PPAR), such as non-alcoholic fatty liver disease (NAFLD) in an individual, which comprises administering a therapeutically effective amount of the crystalline form A, B, C, D or E of the compound of Formula (I), or the amorphous form of the compound of Formula (I) according to any one of 1, 3, 5, 7, 9 and 11, or any combination thereof, to an individual in need thereof,
for example, the disease associated with peroxisome proliferator-activated receptor (PPAR) such as non-alcoholic fatty liver disease (NAFLD) comprises non-alcoholic fatty liver disease (NAFLD).

16. The crystalline form A, B, C, D, E or amorphous form of the compound of Formula (I) according to any one of claims 1, 3, 5, 7,9 and 11, for use in the treatment of a disease associated with peroxisome proliferator-activated receptor (PPAR), such as non-alcoholic fatty liver disease (NAFLD), in an individual,
for example, the disease associated with peroxisome proliferator-activated receptor (PPAR) such as non-alcoholic fatty liver disease (NAFLD) comprises non-alcoholic fatty liver disease (NAFLD).
